# EUROPEAN PATENT APPLICATION

(11) **EP 2 594 651 A1**
(43) Date of publication of application: **22.05.2013**
(21) Application number: 12193119.0
(22) Date of filing: 16.11.2012
(51) Int. Cl.: C12Q 1/68

(54) **Analysis of methylation sites**

(30) Priority: 17.11.2011 GB 201119904
(71) Applicant: Vilnius University, 01513 Vilnius (LT); Centre For Addiction And Mental Health, Toronto, ON M5T 1R8 (CA)
(72) Inventor: Klimasauskas, Saulius, 10220 Vilnius (LT); Kriukiene, Edita, 10311 Vilnius (LT); Urbanaviciute, Giedre, 02241 Vilnius (LT); Petronis, Arturas, Midhurst, Ontario L0L 1X1 (CA); Khare, Tarang, Etobicoke, Ontario M8V 1Y2 (CA); Wang, Sun Chong, New Tapei City (TW)
(74) Representative: Daniels, Jeffrey Nicholas

(57) **Abstract**

The present invention provides a method for labeling unmethylated CpG dinucleotides within a DNA fragment, and use of the method in profiling of genomic DNA methylation. The present invention further provides modified DNA ethyltransferase enzymes and compounds which are capable of being used by the enzymes as cofactors for use in the labeling method.

SEQUENCE LISTING

<110>  Vilnius University et al.

<120>  Analysis of Methylation Sites

<130>  327600EP/CJS

<150>  GB1119904.9
<151>  2011-11-17

<160>  33

<170>  PatentIn version 3.5

<210>  1
<211>  386
<212>  PRT
<213>  Artificial Sequence

<220>
<223>  M.SssI

<400>  1

Met Ser Lys Val Glu Asn Lys Thr Lys Lys Leu Arg Val Phe Glu Ala
1               5                   10                  15

Phe Ala Gly Ile Gly Ala Gln Arg Lys Ala Leu Glu Lys Val Arg Lys
            20                  25                  30

Asp Glu Tyr Glu Ile Val Gly Leu Ala Glu Trp Tyr Val Pro Ala Ile
        35                  40                  45

Val Met Tyr Gln Ala Ile His Asn Asn Phe His Thr Lys Leu Glu Tyr
    50                  55                  60

Lys Ser Val Ser Arg Glu Glu Met Ile Asp Tyr Leu Glu Asn Lys Thr
65                  70                  75                  80

Leu Ser Trp Asn Ser Lys Asn Pro Val Ser Asn Gly Tyr Trp Lys Arg
                85                  90                  95

Lys Lys Asp Asp Glu Leu Lys Ile Ile Tyr Asn Ala Ile Lys Leu Ser
            100                 105                 110

Glu Lys Glu Gly Asn Ile Phe Asp Ile Arg Asp Leu Tyr Lys Arg Thr
        115                 120                 125

Leu Lys Asn Ile Asp Leu Leu Thr Tyr Ser Phe Pro Cys Gln Asp Leu
    130                 135                 140

Ser Gln Gln Gly Ile Gln Lys Gly Met Lys Arg Gly Ser Gly Thr Arg
145                 150                 155                 160

Ser Gly Leu Leu Trp Glu Ile Glu Arg Ala Leu Asp Ser Thr Glu Lys
                165                 170                 175

Asn Asp Leu Pro Lys Tyr Leu Leu Met Glu Asn Val Gly Ala Leu Leu
            180                 185                 190

His Lys Lys Asn Glu Glu Glu Leu Asn Gln Trp Lys Gln Lys Leu Glu
        195                 200                 205

Ser Leu Gly Tyr Gln Asn Ser Ile Glu Val Leu Asn Ala Ala Asp Phe
    210                 215                 220

Gly Ser Ser Gln Ala Arg Arg Arg Val Phe Met Ile Ser Thr Leu Asn
225                 230                 235                 240

Glu Phe Val Glu Leu Pro Lys Gly Asp Lys Lys Pro Lys Ser Ile Lys
                245                 250                 255

Lys Val Leu Asn Lys Ile Val Ser Glu Lys Asp Ile Leu Asn Asn Leu
            260                 265                 270

Leu Lys Tyr Asn Leu Thr Glu Phe Lys Lys Thr Lys Ser Asn Ile Asn
        275                 280                 285

Lys Ala Ser Leu Ile Gly Tyr Ser Lys Phe Asn Ser Glu Gly Tyr Val
    290                 295                 300

Tyr Asp Pro Glu Phe Thr Gly Pro Thr Leu Thr Ala Ser Gly Ala Asn
305                 310                 315                 320

Ser Arg Ile Lys Ile Lys Asp Gly Ser Asn Ile Arg Lys Met Asn Ser
                325                 330                 335

Asp Glu Thr Phe Leu Tyr Ile Gly Phe Asp Ser Gln Asp Gly Lys Arg
            340                 345                 350

Val Asn Glu Ile Glu Phe Leu Thr Glu Asn Gln Lys Ile Phe Val Cys
        355                 360                 365

Gly Asn Ser Ile Ser Val Glu Val Leu Glu Ala Ile Ile Asp Lys Ile
    370                 375                 380

Gly Gly
385

<210>  2
<211>  8
<212>  PRT
<213>  Artificial Sequence

<220>
<223>  M.SssI Subsequence


<220>
<221>  VARIANT
<222>  (5)..(5)
<223>  X is glycine, serine, threonine, asparagine, alanine or valine

<400>  2

Ser Phe Pro Cys Xaa Asp Leu Ser
1               5


<210>  3
<211>  6
<212>  PRT
<213>  Artificial Sequence

<220>
<223>  M.SssI Subsequence


<220>
<221>  VARIANT
<222>  (2)..(2)
<223>  X is glycine, serine, threonine, alanine or valine

<400>  3

Gly Xaa Ser Ile Ser Val
1               5


<210>  4
<211>  358
<212>  PRT
<213>  Artificial Sequence

<220>
<223>  M.HpaII

<400>  4

Met Lys Asp Val Leu Asp Asp Asn Leu Leu Glu Glu Pro Ala Ala Gln
1               5                   10                  15


Tyr Ser Leu Phe Glu Pro Glu Ser Asn Pro Asn Leu Arg Glu Lys Phe
            20                  25                  30


Thr Phe Ile Asp Leu Phe Ala Gly Ile Gly Gly Phe Arg Ile Ala Met
        35                  40                  45


Gln Asn Leu Gly Gly Lys Cys Ile Phe Ser Ser Glu Trp Asp Glu Gln
    50                  55                  60


Ala Gln Lys Thr Tyr Glu Ala Asn Phe Gly Asp Leu Pro Tyr Gly Asp
65                  70                  75                  80

Ile Thr Leu Glu Glu Thr Lys Ala Phe Ile Pro Glu Lys Phe Asp Ile
                85                  90                  95

Leu Cys Ala Gly Phe Pro Cys Gln Ala Phe Ser Ile Ala Gly Lys Arg
            100                 105                 110

Gly Gly Phe Glu Asp Thr Arg Gly Thr Leu Phe Phe Asp Val Ala Glu
        115                 120                 125

Ile Ile Arg Arg His Gln Pro Lys Ala Phe Phe Leu Glu Asn Val Lys
    130                 135                 140

Gly Leu Lys Asn His Asp Lys Gly Arg Thr Leu Lys Thr Ile Leu Asn
145                 150                 155                 160

Val Leu Arg Glu Asp Leu Gly Tyr Phe Val Pro Glu Pro Ala Ile Val
                165                 170                 175

Asn Ala Lys Asn Phe Gly Val Pro Gln Asn Arg Glu Arg Ile Tyr Ile
            180                 185                 190

Val Gly Phe His Lys Ser Thr Gly Val Asn Ser Phe Ser Tyr Pro Glu
        195                 200                 205

Pro Leu Asp Lys Ile Val Thr Phe Ala Asp Ile Arg Glu Glu Lys Thr
    210                 215                 220

Val Pro Thr Lys Tyr Tyr Leu Ser Thr Gln Tyr Ile Asp Thr Leu Arg
225                 230                 235                 240

Lys His Lys Glu Arg His Glu Ser Lys Gly Asn Gly Phe Gly Tyr Glu
                245                 250                 255

Ile Ile Pro Asp Asp Gly Ile Ala Asn Ala Ile Val Val Gly Gly Met
            260                 265                 270

Gly Arg Glu Arg Asn Leu Val Ile Asp His Arg Ile Thr Asp Phe Thr
        275                 280                 285

Pro Thr Thr Asn Ile Lys Gly Glu Val Asn Arg Glu Gly Ile Arg Lys
    290                 295                 300

Met Thr Pro Arg Glu Trp Ala Arg Leu Gln Gly Phe Pro Asp Ser Tyr
305                 310                 315                 320

Val Ile Pro Val Ser Asp Ala Ser Ala Tyr Lys Gln Phe Gly Asn Ser
                325                 330                 335

Val Ala Val Pro Ala Ile Gln Ala Thr Gly Lys Lys Ile Leu Glu Lys

340                 345                 350

Leu Gly Asn Leu Tyr Asp
        355

<210>  5
<211>  8
<212>  PRT
<213>  Artificial Sequence

<220>
<223>  M.HpaII Subsequence

<220>
<221>  VARIANT
<222>  (5)..(5)
<223>  X is glycine, serine, threonine, asparagine, alanine or valine

<400>  5

Gly Phe Pro Cys Xaa Ala Phe Ser
1               5

<210>  6
<211>  6
<212>  PRT
<213>  Artificial Sequence

<220>
<223>  M.HpaII Subsequence

<220>
<221>  VARIANT
<222>  (2)..(2)
<223>  X is glycine, serine, threonine, alanine or valine

<400>  6

Gly Xaa Ser Val Ala Val
1               5

<210>  7
<211>  327
<212>  PRT
<213>  Artificial Sequence

<220>
<223>  M.HhaI

<400>  7

Met Ile Glu Ile Lys Asp Lys Gln Leu Thr Gly Leu Arg Phe Ile Asp
1               5                   10                  15

Leu Phe Ala Gly Leu Gly Gly Phe Arg Leu Ala Leu Glu Ser Cys Gly
            20                  25                  30

Ala Glu Cys Val Tyr Ser Asn Glu Trp Asp Lys Tyr Ala Gln Glu Val
        35                  40                  45

Tyr Glu Met Asn Phe Gly Glu Lys Pro Glu Gly Asp Ile Thr Gln Val
    50                  55                  60

Asn Glu Lys Thr Ile Pro Asp His Asp Ile Leu Cys Ala Gly Phe Pro
65                  70                  75                  80

Cys Gln Ala Phe Ser Ile Ser Gly Lys Gln Lys Gly Phe Glu Asp Ser
                85                  90                  95

Arg Gly Thr Leu Phe Phe Asp Ile Ala Arg Ile Val Arg Glu Lys Lys
            100                 105                 110

Pro Lys Val Val Phe Met Glu Asn Val Lys Asn Phe Ala Ser His Asp
        115                 120                 125

Asn Gly Asn Thr Leu Glu Val Val Lys Asn Thr Met Asn Glu Leu Asp
    130                 135                 140

Tyr Ser Phe His Ala Lys Val Leu Asn Ala Leu Asp Tyr Gly Ile Pro
145                 150                 155                 160

Gln Lys Arg Glu Arg Ile Tyr Met Ile Cys Phe Arg Asn Asp Leu Asn
                165                 170                 175

Ile Gln Asn Phe Gln Phe Pro Lys Pro Phe Glu Leu Asn Thr Phe Val
            180                 185                 190

Lys Asp Leu Leu Leu Pro Asp Ser Glu Val Glu His Leu Val Ile Asp
        195                 200                 205

Arg Lys Asp Leu Val Met Thr Asn Gln Glu Ile Glu Gln Thr Thr Pro
    210                 215                 220

Lys Thr Val Arg Leu Gly Ile Val Gly Lys Gly Gly Gln Gly Glu Arg
225                 230                 235                 240

Ile Tyr Ser Thr Arg Gly Ile Ala Ile Thr Leu Ser Ala Tyr Gly Gly
                245                 250                 255

Gly Ile Phe Ala Lys Thr Gly Gly Tyr Leu Val Asn Gly Lys Thr Arg
            260                 265                 270

Lys Leu His Pro Arg Glu Cys Ala Arg Val Met Gly Tyr Pro Asp Ser
        275                 280                 285

Tyr Lys Val His Pro Ser Thr Ser Gln Ala Tyr Lys Gln Phe Gly Asn

290                 295                 300

Ser Val Val Ile Asn Val Leu Gln Tyr Ile Ala Tyr Asn Ile Gly Ser
305                 310                 315                 320

Ser Leu Asn Phe Lys Pro Tyr
                325

<210>  8
<211>  8
<212>  PRT
<213>  Artificial Sequence

<220>
<223>  M.HhaI Subsequence

<220>
<221>  VARIANT
<222>  (5)..(5)
<223>  X is glycine, serine, threonine, asparagine, alanine or valine

<400>  8

Gly Phe Pro Cys Xaa Ala Phe Ser
1               5

<210>  9
<211>  6
<212>  PRT
<213>  Artificial Sequence

<220>
<223>  M.HhaI Subsequence

<220>
<221>  VARIANT
<222>  (2)..(2)
<223>  X is glycine, serine, threonine, alanine or valine

<400>  9

Gly Xaa Ser Val Val Ile
1               5

<210>  10
<211>  17
<212>  DNA
<213>  Artificial Sequence

<220>
<223>  Primer I

<400>  10
gtcctggcca cgggtgc                                                    17

<210>  11
<211>  20

<212>  DNA
<213>  Artificial Sequence

<220>
<223>  Primer II

<400>  11
tccgcgtttc cagactttac                                                    20


<210>  12
<211>  17
<212>  DNA
<213>  Artificial Sequence

<220>
<223>  Primer III

<400>  12
gtcgttcggc tgcggcg                                                       17


<210>  13
<211>  21
<212>  DNA
<213>  Artificial Sequence

<220>
<223>  Primer IV

<400>  13
tgacttgagc gtcgattttt g                                                  21


<210>  14
<211>  20
<212>  DNA
<213>  Artificial Sequence

<220>
<223>  Primer V

<400>  14
gtgttggggt gactattatg                                                    20


<210>  15
<211>  18
<212>  DNA
<213>  Artificial Seqeuence

<220>
<223>  Primer VI

<400>  15
cctatactca gcgcatcc                                                      18


<210>  16
<211>  19
<212>  DNA
<213>  Artificial Seqeuence

<220>
<223>  Primer VII

<400>  16
gcccacttca cttcttgtg                                                    19


<210>  17
<211>  21
<212>  DNA
<213>  Artificial Sequence

<220>
<223>  Primer VIII

<400>  17
aggccaaaag aaagaagaga t                                                 21


<210>  18
<211>  20
<212>  DNA
<213>  Artificial Sequence

<220>
<223>  Primer Specific-dir

<400>  18
gggttgcctt actggttagc                                                   20


<210>  19
<211>  20
<212>  DNA
<213>  Artificial Sequence

<220>
<223>  Primer Specific-rev

<400>  19
tccgcgtttc cagactttac                                                   20


<210>  20
<211>  23
<212>  DNA
<213>  Artificial Sequence

<220>
<223>  Primer TaqMan probe


<220>
<221>  misc_feature
<222>  (1)..(1)
<223>  Label

<220>
<221>  misc_feature
<222>  (23)..(23)
<223>  Label

<400>  20
atgaatcacc gatacgcgag cga                                               23


<210>  21

<211>  20
<212>  DNA
<213>  Artificial Sequence

<220>
<223>  Primer Nonspecific-dir

<400>  21
agctcactca aaggcggtaa                                                   20


<210>  22
<211>  20
<212>  DNA
<213>  Artificial Sequence

<220>
<223>  Primer Nonspecific-rev

<400>  22
tttttgtgat gctcgtcagg                                                   20


<210>  23
<211>  24
<212>  DNA
<213>  Artificial Sequence

<220>
<223>  Primer TaqMan probe


<220>
<221>  misc_feature
<222>  (1)..(1)
<223>  Label

<220>
<221>  misc_feature
<222>  (24)..(24)
<223>  Label

<400>  23
aaggccagga accgtaaaaa ggcc                                              24


<210>  24
<211>  20
<212>  DNA
<213>  Artificial Sequence

<220>
<223>  Specific-dir

<400>  24
atgtgttgga gtgtgcctga                                                   20


<210>  25
<211>  20
<212>  DNA
<213>  Artificial sequence

<220>
<223>  Primer Specific-rev

<400>  25
gtggctctga ttgatggctc                                                    20


<210>  26
<211>  26
<212>  DNA
<213>  Artificial Sequence

<220>
<223>  Primer TaqMan probe


<220>
<221>  misc_feature
<222>  (1)..(1)
<223>  Label

<220>
<221>  misc_feature
<222>  (26)..(26)
<223>  Label

<400>  26
tccctgtgtg atcaccccta tgcttg                                             26


<210>  27
<211>  19
<212>  DNA
<213>  Artificial Sequence

<220>
<223>  Primer Nonspecific-dir

<400>  27
caggcctctt caagggtca                                                     19


<210>  28
<211>  18
<212>  DNA
<213>  Artificial Sequence

<220>
<223>  Primer Nonspecific-rev

<400>  28
aagagatgag ggcctggg                                                      18


<210>  29
<211>  23
<212>  DNA
<213>  Artificial Sequence

<220>
<223>  Primer TaqMan probe


<220>
<221>  misc_feature
<222>  (1)..(1)
<223>  Label

<220>
<221>  misc_feature
<222>  (26)..(26)
<223>  Label

<400>  29
tggcccatac ctcttcaagg gca                                               23


<210>  30
<211>  25
<212>  DNA
<213>  Artificial Sequence

<220>
<223>  Oligonucleotide IX

<400>  30
agttacatct tgtagtcagt ctcca                                             25


<210>  31
<211>  19
<212>  DNA
<213>  Artificial Sequence

<220>
<223>  Oligonucleotide X

<400>  31
tggagactga ctacaagat                                                    19


<210>  32
<211>  24
<212>  DNA
<213>  Artificial Sequence

<220>
<223>  Oligonucleotide

<400>  32
gcattacgcg ccaggtcgtt tcgt                                              24


<210>  33
<211>  24
<212>  DNA
<213>  Artificial Sequence

<220>
<223>  Oligonucleotide

<400>  33
tacgaaacga cctggcgcgt aatg                                              24

## Description

All or part of the work performed during the development of this invention utilized U.S. Government funds, Accordingly, the U.S. Government has a paid-up license in this invention and the right in limited circumstances to require the patent owner to license other on reasonable terms as provided for by the terms of Grant No. MH074127; MH088413; DP3DK085698; HG004535 awarded by the National Institutes of Health.

The present invention relates to methods associated with the analysis or interrogation of methylation sites within DNA molecules. The invention is also concerned with reaction components suitable for use in these methods.

### Background to the invention

Genomic DNA methylation is a key epigenetic regulatory mechanism in high eukaryotes. DNA methylation profiles (occurrence of methylated cytosines) are highly variable across different genetic loci, cells and organisms, and are dependent on tissue, age, sex, diet, and other factors. Aberrant DNA methylation correlates with a number of pediatric syndromes and cancer, or predisposes individuals to various other human diseases. However, research into the epigenetic misregulation and its diagnostics is hampered by the lack of adequate analytical techniques. A myriad of techniques exist for the identification of methylated cytosines. There are now numerous technologies available to interrogate the DNA methylation status of CpG sites in a targeted or genome-wide fashion, but each method, due to intrinsic biases, potentially interrogates different fractions of the genome. Most of the analytical approaches can be divided into bisulfite-based methods, the enrichment-based techniques and digestion with methylation-sensitive restriction enzymes. All these approaches can be used in conjunction with microarray analysis or massively parallel sequencing to map DNA methylation on a genomic scale. Since all available high-throughput methods have their strengths and weaknesses, no universal method exists which suits best to answer all epigenetic questions.

Bisulfite modification has been the gold standard technique in DNA methylation analysis (Frommer et al. PNAS, 1992, 89, 1827-1831). Sodium bisulfite converts unmethylated cytosines (C) into uracils, which become thymines during PCR, while ^{met}C are protected and do not change. The key advantage of this method is sensitivity, because the technology allows the high resolution to a single nucleotide analysis and an ability to quantify methylation levels. While the approach is very informative and quite precise, the genome-wide bisulfite sequencing is one of the most labour and cost intensive techniques in the field of epigenetics.

The enrichment-based technologies for interrogation of methylated DNA regions use methyl-DNA immunoprecipitation (MeDIP) that is based on enrichment with antibodies specific for 5'-methylcytosine (metC) or the capture of methylated DNA using a methyl-CpG binding domain protein (MBD). Both methods are able to provide broad coverage of the genome, though are also subject to some limitations. (Robinson et al., Genome Res., 2010, 20, 1719-1729; Nair, et al., Epigenetics, 2011, 6, 34-44). Both enrichment techniques are sensitive for detecting differently methylated regions, with MeDIP commonly enriching for methylated regions with a low CpG density, while MBD capture favors regions of higher CpG density and identifies the greatest proportion of CpG islands. Although enrichment methods provide lower cost per CpG covered relative to bisulfite-methods, they do not allow precise quantification of methylation level and are largely dependent on CpG density. Beside sensitivity to CpGs density, the affinity-enrichment methods are prone to amplification bias, and copy number variation (Robinson et al., Genome Res., 2010, 20, 1719-1729).

Methylation sensitive restriction enzymes were first applied to epigenetic studies over three decades ago and, for many years, were the primary tools for DNA methylation analysis, until the development of the fine mapping using bisulfite modification approaches. A variety of restriction enzymes are available for large-scale DNA methylation profiling using microarrays or next generation sequencing. Microarrays provide a significant advancement for the methylation analysis of complex genomes, because they can interrogate a very large number of loci in a highly parallel fashion. Whereas next-generation sequencing enables higher resolution and higher genomic coverage in comparison to microarrays, microarray analysis is still more cost efficient and an excellent approach when exploring methylation changes that occur in diseases phenotypes or searching for potential diagnostic biomarkers.

Generally, the sequence specificity of restriction endonucleases is the major limitation of this approach. Restriction enzyme-based approach allows for interrogation of either the unmethylated or methylated fraction of genomic DNA. Most restriction enzyme - based epigenomic profiling studies have been performed using the methylated fraction of genomic DNA (Huang et al. Hum Mol Genet 1999, 8, 459-470; Hatada et al. 2002, J Hum Genet 47, 448-451; Yan et al. 2002, Methods 27, 162-169; Shi et al. Cancer Res, 2003, 63, 2164-2171). While the focus on the methylated genome is in some cases justified and beneficial (e.g. identification of de novo methylated CpG islands in cancer), the interrogation of the unmethylated DNA fraction could be more efficient than analysing the hypermethylated fraction of the genome (Schumacher et al., Nucleic Acids Res, 2006, 34, 528-542). This is based on the observation that unmethylated cytosines represent a much smaller proportion of cytosines compared to methylated ones (depending on the tissue, over 70% of cytosines in the human genome are methylated). Analysis of this smaller unmethylated fraction is more sensitive to detect subtle methylation abnormalities. For example, if 20% of all CpGs in a given tissue are unmethylated, a de novo methylation of 10% would result in 100% (decrease of from 20% to 10%) difference in the unmethylated fraction. In the same scenario, only a 12% change (from 80% to 90%) would be detected for the hypermethylated fraction of genomic DNA.

The genomic CpG coverage of the restriction endonuclease-based method is limited by sequence-specificity of the enzymes used for cleavage of genomic DNA. The combination of the three commonly used enzymes, HpaII, Hin6I and AciI, interrogates ~32% of all CpG dinucleotides in mammalian DNA (Schumacher et al. Nucleic Acids Res, 2006, 34, 528-542). The application of more restriction enzymes might be disadvantageous for the analysis of CpG rich regions as such a strategy would produce restriction fragments too short for analysis on microarrays. Therefore, for analysis of methylation levels of a single CpG dinucleotide in the genome, new methods are required that employ the enzymes with reduced sequence specificity.

It is an aim of the present invention to solve one or more of the problems with the prior art.

### Summary of the invention

Accordingly, the present invention provides a method for labeling unmethylated CpG dinucleotides within a DNA fragment, said method comprising the steps of:
(a)
   i) modifying the DNA fragment at the unmethylated CpG dinucleotide by contacting the DNA fragment with a mutant C5-methyltransferase enzyme and a co-factor under conditions which allow for the transfer of a part of the co-factor onto the unmethylated CpG dinucleotide to form a modified CpG dinucleotide; and
   (ii) contacting the modified CpG dinucleotide with a compound comprising a label under conditions which allow for the transfer of the label to the modified CpG dinucleotide to form a labeled DNA fragment; or
(b) modifying the DNA fragment at the unmethylated CpG dinucleotide by contacting the DNA fragment with a mutant C5-methyltransferase enzyme and a co-factor comprising a label under conditions which allow for the transfer of the label onto the unmethylated CpG dinucleotide to form a labeled DNA fragment,
wherein the mutant C-5 methyltransferase enzyme has an amino acid sequence which comprises a glycine, serine, threonine, asparagine, alanine or valine in place of the conserved glutamine residue in motif IV and a glycine, serine, threonine, alanine or valine in place of the conserved asparagine residue in motif X,
and wherein, when the mutant C-5 methyltransferase enzyme comprises M.HhaI having an amino acid sequence which comprises the mutations Q32A and N304A, the DNA fragment is labeled using more than one mutant C-5 methyltransferase enzymes.

Further, the present invention provides a method for analyzing unmethylated CpG dinucleotides within one or more DNA molecules, comprising the steps of:
(a) providing fragments of the DNA molecules;
(b) labeling the unmethylated CpG dinucleotides using the method of the above paragraph to produce labeled DNA fragments;
(c) enriching the labeled DNA fragments;
(d) amplifying the enriched labeled DNA fragments; and
(e) analyzing the amplified DNA fragments to determine the methylation status of the CpG dinucleotides.

The present invention provides a new approach to genomic DNA profiling which makes use of the DNA methyltransferase-directed transfer of functional groups from synthetic cofactors based on S-adenosyl-L-methionine (SAM or AdoMet) (the so-called mTAG technology, described in Lukinavicius et al. J. Am. Chem. Soc. 2007, 129, 2758-2759, and WO2006/108678) in combination with microarray-based DNA methylation profiling or parallel sequencing techniques.

The technological innovation of mTAG consists of labeling unmethylated cytosines using synthetic AdoMet cofactors. The present invention extends this technology through the identification of mutant DNA methyltransferases, and further synthetic AdoMet cofactors, which allow the efficient labeling and separation of DNA fragments containing unmethylated CpG dinucleotides from the bulk of genomic DNA, so that the fragments can be interrogated on tiling microarrays. Accordingly, the present invention enables the use of mTAG technology in genome methylation profiling.

The new technology permits distinction of every unmethylated CG site in any genome and demonstrates the advantages of using the unmethylated DNA fraction versus methylated one (Schumacher et al., Nucleic Acids Res. 2006, 34, 528-542).

The present invention also provides mutant DNA methyltransferases and synthetic AdoMet based cofactors for use in the above described method.

Protein engineering approaches were used to construct novel mutants of C5 DNA methyltransferase enzymes which target cytosine in the CpG context in their recognition sites, and are surprisingly useful in the methods of the present invention.

In particular, the present invention provides a mutant CpG C-5 methyltransferase enzyme, said enzyme having an amino acid sequence which comprises glycine, serine, threonine, asparagine, alanine or valine in place of the conserved glutamine residue in motif IV and a glycine, serine, threonine, alanine or valine in place of the conserved asparagine residue in motif X, wherein said enzyme is not M.HhaI.

These mutant CpG C-5 methyltransferase enzyme, such as M.HpaII (CCGG target site) and M.SssI (CG target site) showed surprisingly enhanced transalkylation activity with synthetic cofactors.

The present invention further provides a polynucleotide which encodes the CpG methyltransferase of the above paragraphs. Such a polynucleotide can be used to produce the CpG methyltransferase. In particular, a method for producing the CpG methyltransferase is provided which comprises expressing the polynucleotide of the invention.

Further, the present invention provides a compound represented by formula (I): 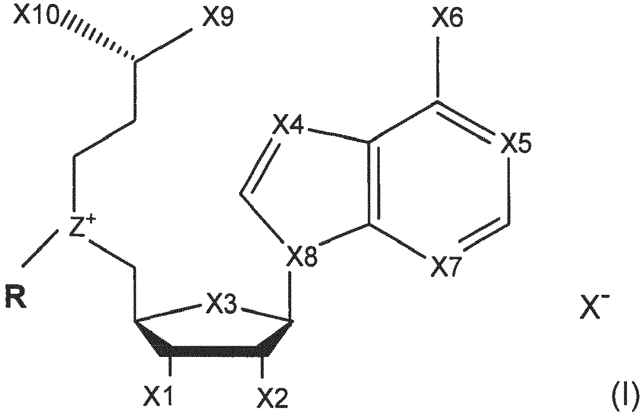 where
X1 and X2 represent -OH, -NH₂, -SH, -H or-F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH,, -OCH₃, -H, -F, -Cl, -SH or -NHCH₃, and preferably is -NH₂;
X9 represents -CO₂H, -PO₃H, -H, -CHO, -CH₃, or -CH₂OH and preferably is -CO₂H;
X10 represents -NH₂, -OH, -H, -CH₃, or -NHCH₃, and preferably is -NH₂;
X⁻ is an organic or inorganic anion selected from trifluoroacetate, formate, halide and sulfonate;
Z represents S or Se, and preferably is S;
C-bound H atoms in the adenosine moiety can be replaced by -F, -OH, -NH₂, or -CH₃, but are preferably H;
R comprises -CH=CH- or -C=C- in a β-position to Z+ centre and separated therefrom by CR1R2-, where R1 and R2 are independently H or D;
R further comprises a functional group selected from an amino group, a thiol group, a 1,2-diol group, a hydrazine group, a hydroxylamine group, a 1,2-aminothiol group, an azide group, a diene group, an alkyne group, an arylhalide group, a terminal silylalkyne group, an N-hydroxysuccinimidyl ester group, a thioester group, an isothiocyanate group, an imidoester group, a maleimide group, a haloacetamide group, an aziridine group, an arylboronic acid group, an aldehyde group, a ketone group, a phosphane ester group, a dienophile group, a terminal haloalkyne group,
wherein the distance between -CH=CH- or -C=C- in the β-position to Z+ centre and the functional group is no more than 7 atoms in length,
and wherein the distance between -CH=CH- or -C≡C- and the nearest electronegative atom or group in R is at least 2 carbon atoms.

The inventors have found new suitable cofactor analogs and elaborated a synthetic pathway for preparing these in suitable quantities. The cofactor analogues are surprisingly useful in combination with the mutant DNA methyltransferases enzyme of the present invention. In particular, the combination in the method of profiling results in only a low level of off-target methylation, efficient labeling of the modified DNA molecule and efficient enrichment and amplification of the labeled DNA molecules.

Accordingly, the present invention further provides a method of producing a compound according to formula (I) above comprising a step of reacting an activated compound comprising R with a compound of formula (IV) under conditions which allow the R group to be coupled to the Z of the compound of formula (IV), wherein formula (IV) is: 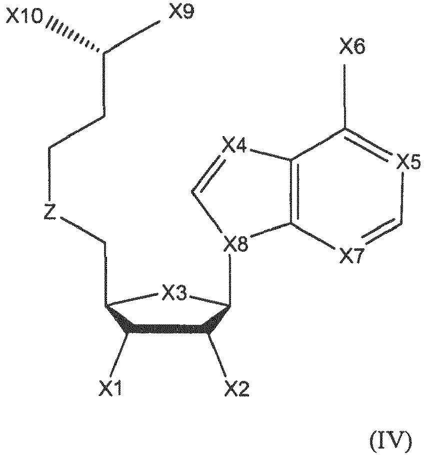 where
X1 and X2 represent -OH, -NH₂, -SH, -H or -F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH,, -OCH₃, -H, -F, -Cl, -SH or -NHCH₃, and preferably is -NH₂;
X9 represents -CO₂H, -PO₃H, -H, -CHO, -CH₃, or -CH₂OH and preferably is -CO₂H;
X10 represents -NH₂, -OH, -H, -CH₃, or -NHCH₃, and preferably is -NH₂;
X⁻ is an organic or inorganic anion selected from trifluoroacetate, formate, halide and sulfonate;
Z represents S or Se, and preferably is S;
C-bound H atoms in the adenosine moiety can be replaced by -F, -OH, -NH₂, or -CH₃, but are preferably H;

The present invention also provides kits comprising the above compound of formula (I), preferably with one or more of the enzymes described above, and kits comprising more than one of the above described mutant enzymes.

In a further aspect the present invention provides a complex of the above compound of formula (I) with a methyltransferase which is capable of using S-adenosyl-L-methionine as a cofactor.

In a still further aspect the present invention provides uses of the compounds, enzymes and kits described above. In particular, the present invention provides use of the above described compound, methyltransferase enzyme or kit for modifying a target molecule, such as a nucleic acid molecule, a polypeptide, a carbohydrate or a small molecule, such as a phospholipid, an amino acid, a hormone, a nucleotide, a nucleoside or a derivative thereof. Preferably the target molecule is DNA.

The present invention further provides a nucleic acid molecule derivatised by a methyltransferase using the compound of formula (I) described above.

### Figures

Figure 1: Flow chart of an embodiment of the invention using mTAG labeling-based analysis of the unmethylated fraction of a genome.
Figure 2: Structure and general synthetic route to Ado-6-amine and Ado-11-amine cofactors via 6-[(tret-Butoxycarbonylamino)butanamido]hex-2-in-1-ol.

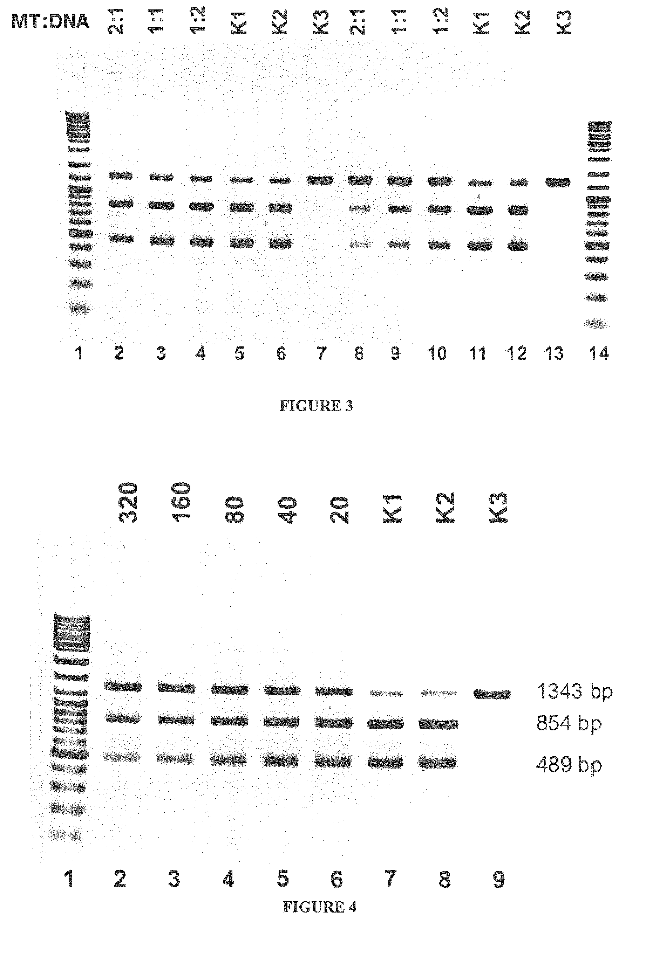

Figure 3: Enzymatic activity of M.SssI (His6 Q142A/N370A mutant) with cofactor Ado-6-amine in the reaction buffer (10 mM Tris-HCl (pH7.5), 50 mM NaCl, 0.1 mg/ml) in the presence (Lanes 2-7) or absence (Lanes 8-13) of 10 mM MgCl₂. **Lanes 1, 14** Molecular mass standard GeneRuler™ DNA Ladder Mix; **Lanes 2-4** 1343bp DNA + 40 µM cofactor + M.SssI + R.Hin6I; **Lane 5-** control line, DNA + R.Hin6I; **Lane 6-** control line DNA + M.SssI + R.Hin6I; 7- control, untreated DNA; **Lanes 8-10** DNA + 40 µM cofactor+ M.SssI + R.Hin6I, **11**- control line, DNA + R.Hin6I; **Lane 12-** control line DNA + M.SssI + R.Hin6I; **Lane 13-** control, untreated DNA; The molar ratios of M.SssI to CG target sites (MT:DNA) are indicated above the photograph.
Figure 4: Transalkylation activity of M.SssI (His6 Q142A/N370A variant) in the presence of various amounts of the cofactor Ado-11-amine. **Lane 1**, Molecular mass standard GeneRuler™ DNA Ladder Mix (Fermentas). **Lanes 2-6**, DNA + cofactor + M.SssI + R.Hin6I; **Lane 7-** control lane, DNA + R.Hin6I; **Lane 8-** control lane, DNA + MTase + R.Hin6I; **Lane 9-** control, untreated 1343 bp DNA. MTase to DNA molar ratio is 3.6:1.
Figure 5: Identity of modification product formed in DNA upon action of M.SssI (His6 Q142A/N370A mutant) with cofactor Ado-6-amine.
Figure 6: Structure and general synthetic route to the cofactor Ado-biotin.
Figure 7: Enzymatic activity of M.HhaI with cofactor Ado-biotin.
Figure 8: Efficiency of M.HpaII-directed labelling of model DNA fragments.
Figure 9: M.HhaI-directed labelling and enrichment of genomic DNA fragments.
Figure 10: M.SssI-directed labelling and enrichment of genomic DNA fragments.
Figure 11: Recovery of mTAG labelled DNA from streptavidin coated magnetic beads.
Figure 12: Concordance of the mTAG and meDIP data with bisulfitome (http://neomorph.salk.edu/human methylome/data.html) in human chromosome 15 (Lister et al., Nature, 2009, 462, 315-322).
Figure 13: Pearson correlations of mTAG-based (labelling efficiency of 25%) analysis and meDIP based analysis of methylation across 10 deciles of CG density with bisulfitome data of human chromosome 4 (Lister et al., Nature, 2009, 462, 315-322).

### Detailed description of the invention

As indicated above, the present invention provides a method for the labeling of unmethylated CpG dinucleotides in DNA fragments, and subsequent enrichment procedures based on the label, which are particularly suitable for use in the context of methods for profiling of genomic methylation patterns.

In a first aspect the present invention provides a method for labeling unmethylated CpG dinucleotides within a DNA fragment, said method comprising the steps of:
(a)
   (i) modifying the DNA fragment at the unmethylated CpG dinucleotide by contacting the DNA fragment with a mutant C5-methyltransferase enzyme and a co-factor under conditions which allow for the transfer of a part of the co-factor onto the unmethylated CpG dinucleotide to form a modified CpG dinucleotide; and
   (ii) contacting the modified CpG dinucleotide with a compound comprising a label under conditions which allow for the transfer of the label to the modified CpG dinucleotide to form a labeled DNA fragment; or
(b) modifying the DNA fragment at the unmethylated CpG dinucleotide by contacting the DNA fragment with a mutant C5-methyltransferase enzyme and a co-factor comprising a label, under conditions which allow for the transfer of the label onto the unmethylated CpG dinucleotide to form a labeled DNA fragment,
wherein the mutant C-5 methyltransferase enzyme has an amino acid sequence which comprises a glycine, serine, threonine, asparagine, alanine or valine in place of the conserved glutamine residue in motif IV and a glycine, serine, threonine, alanine or valine in place of the conserved asparagine residue in motif X,
and wherein, when the mutant C-5 methyltransferase enzyme comprises M.HhaI having an amino acid sequence which comprises the mutations Q32A and N304A, the DNA fragment is labeled using more than one mutant C-5 methyltransferase enzymes.

In particular, one embodiment of this aspect of the invention is a method for labeling unmethylated CpG dinucleotides within a DNA fragment comprising modifying the DNA fragment at the unmethylated CpG dinucleotide by contacting the DNA fragment with a C5-methyltransferase enzyme and a co-factor comprising a label, under conditions which allow for the transfer of the label onto the unmethylated CpG dinucleotide by the C5-methyltransferase enzyme to form a labeled DNA fragment comprising a CpG dinucleotide modified with the label, wherein the mutant C-5 methyltransferase enzyme has an amino acid sequence which comprises a glycine, serine, threonine, asparagine, alanine or valine in place of the conserved glutamine residue in motif IV and a glycine, serine, threonine, alanine or valine in place of the conserved asparagine residue in motif X,
and wherein, when the mutant C-5 methyltransferase enzyme comprises M.Hhal having an amino acid sequence which comprises the mutations Q32A and N304A, the DNA fragment is labeled using more than one mutant C-5 methyltransferase enzymes.

An alternative embodiment of this aspect of the invention is a method for labeling unmethylated CpG dinucleotides within a DNA fragment comprising:
(i) modifying the DNA fragment at the unmethylated CpG dinucleotide by contacting the DNA fragment with a mutant C5-methyltransferase enzyme and a co-factor under conditions which allow for the transfer of a part of the co-factor onto the unmethylated CpG dinucleotide by the C5-methyltransferase enzyme to form a modified CpG dinucleotide; and
(ii) contacting the modified CpG dinucleotide with a compound comprising a label under conditions which allow for the transfer of the label to the modified CpG dinucleotide to form a labeled DNA fragment,
wherein the mutant C-5 methyltransferase enzyme has an amino acid sequence which comprises a glycine, serine, threonine, asparagine, alanine or valine in place of the conserved glutamine residue in motif IV and a glycine, serine, threonine, alanine or valine in place of the conserved asparagine residue in motif X,
and wherein, when the mutant C-5 methyltransferase enzyme comprises M.HhaI having an amino acid sequence which comprises the mutations Q32A and N304A, the DNA fragment is labeled using more than one mutant C-5 methyltransferase enzymes.

The strategy of utilising DNA methyltransferase enzymes to transfer groups from synthetic co-factors (cofactor analogues) onto unmethylated cytosines residue in a DNA molecule based on the enzyme's recognition site are described in the art (Lukinavicius et al. J. Am. Chem. Soc. 2007, 129, 2758-2759, and WO2006/108678). In particular, the enzymes usually transfer methyl groups from the co-factor S-adenoyl-L-methionine (SAM or AdoMet) onto various positions in the DNA sequence. However, the enzymes are also able to transfer other groups from synthetic AdoMet analogues, enabling a labeling procedure, as described in WO2006/108678.

As indicated above, the method of the first aspect of the invention utilizes C-5 methyltransferase enzymes. Accordingly, in a related second aspect a mutant C5-methyltransferase enzyme is provided, said enzyme having an amino acid sequence which comprises glycine, serine, threonine, asparagine, alanine or valine in place of the conserved glutamine residue in motif IV and a glycine, serine, threonine, alanine or valine in place of the conserved asparagine residue in motif X, wherein said enzyme is not M.HhaI.

In particular, the mutant enzyme is a mutant form of a C5 methyltransferase, where a C5 methyltransferase is an enzyme which, in non-mutant form, is capable of methylating the 5-carbon of the pyrimidine ring of cytosine, using the co-factor S-adenoyl-L-methionine, to create 5-methylcytosine. Many C5 methyltransferase enzymes are known in the art and are known to have ten conserved motifs, motif I to motif X (Kumar et al., Nucleic Acids Research, 1994, Vol 22, No. 1, pages 1 to 10). In particular, motif IV and motif X are among those which are highly conserved.

In the context of the present invention a "mutant" C5-methyltransferase enzyme is one which has an amino acid sequence which comprises a mutation of the conserved glutamine residue in motif IV (which usually is found within the sequence PCQ) and the conserved asparagine residue in motif X (which is usually found within the sequence GNS/A).

Suitable C5 methyltransferases, on which the mutants of the present invention can be based, are known in the art and in particular are listed in the REBASE database available at http://rebase.neb.com/rebase/rebase.html.

The mutant enzymes of the present invention can be made using recombinant techniques which are well known in the art. The present invention also provides nucleic acid sequence encoding the enzymes of the invention which can be used in the production of these mutant enzymes. In particular, the nucleic acids sequences can be isolated nucleic acid sequences, or part of a vector, such as a plasmid. The nucleic acid sequences can be used in expression vectors to produce the enzymes. Such a method can comprise culturing host cells comprising the expression vectors in vitro under conditions which allow for the nucleic acid sequence expression, and collecting the expressed proteins.

Accordingly, the present invention further provides a method of producing a mutant CpG C-5 methyltransferase as described herein comprising expressing the polynucleotide encoding the same described herein.

In preferred embodiments the mutant C-5 methyltransferase enzyme is an M.SssI enzyme having an amino acid sequence which comprises the mutations at conserved residues Q142 and N370 such that Q142 is replaced by a glycine, serine, threonine, asparagine, alanine or valine, and N370 is replaced by a glycine, serine, threonine, alanine or valine. In this embodiment, the M.SssI enzyme can be additionally defined as having an amino acid sequence which comprises SEQ ID No: 2 and SEQ ID No: 3, and/or having an amino acid sequence which is at least 85%, more preferably at least 90% or 95%, identical to SEQ ID No: 1. Still more preferably the enzyme is one in which Q142 and N370 are replaced by alanine. 
SEQ ID No: 1

MSKVENKTKK LRVFEAFAGI GAQRKALEKV RKDEYEIVGL AEWYVPAIVM YQAIHNNFHT
KLEYKSVSRE EMIDYLENKT LSWNSKNPVS NGYWKRKKDD ELKIIYNAIK LSEKEGNIFD
IRDLYKRTLK NIDLLTYSFP CQDLSQQGIQ KGMKRGSGTR SGLLWEIERA LDSTEKNDLP
KYLLMENVGA LLHKKNEEEL NQWKQKLESL GYQNSIEVLN AADFGSSQAR RRVFMISTLN
EFVELPKGDK KPKSIKKVLN KIVSEKDILN NLLKYNLTEF KKTKSNINKA SLIGYSKFNS
EGYVYDPEFT GPTLTASGAN SRIKIKDGSN IRKMNSDETF LYIGFDSQDG KRVNEIEFLT
ENQKIFVCGN SISVEVLEAI IDKIGG
 SEQ ID No: 2: SFPCXDLS where X is glycine, serine, threonine, asparagine, alanine or valine
SEQ ID No: 3: GXSISV wherein X is glycine, serine, threonine, alanine or valine

In a further preferred embodiment the mutant CpG C-5 methyltransferase enzyme is M.Hpa II enzyme having an amino acid sequence which comprises the mutations at conserved residues Q104 and N3 3 5 such that Q104 is replaced by a glycine, serine, threonine, asparagine, alanine or valine, and N335 is replaced by a glycine, serine, threonine or valine. In this embodiment the M.Hpa II enzyme can be additionally defined as having an amino acid sequence which comprises SEQ ID No: 5 and SEQ ID No: 6, and/or having an amino acid sequence which is at least 85%, more preferably at least 90% or 95%, identical to SEQ ID No: 4. Still more preferably the enzyme is one in which Q104 and N335 are replaced by alanine. 
SEQ ID No: 4

MKDVLDDNLL EEPAAQYSLF EPESNPNLRE KFTFIDLFAG IGGFRIAMQN LGGKCIFSSE
 
WDEQAQKTYE ANFGDLPYGD ITLEETKAFI PEKFDILCAG FPCQAFSIAG KRGGFEDTRG
TLFFDVAEII RRHQPKAFFL ENVKGLKNHD KGRTLKTILN VLREDLGYFV PEPAIVNAKN
FGVPQNRERI YIVGFHKSTG VNSFSYPEPL DKIVTFADIR EEKTVPTKYY LSTQYIDTLR
KHKERHESKG NGFGYEIIPD DGIANAIVVG GMGRERNLVI DHRITDFTPT TNIKGEVNRE
GIRKMTPREW ARLQGFPDSY VIPVSDASAY KQFGNSVAVP AIQATGKKIL EKLGNLYD
 SEQ ID No: 5 GFPCXAFS where X is glycine, serine, threonine, asparagine, alanine or valine
SEQ ID No: 6 GXSVAV wherein X is glycine, serine, threonine, alanine or valine

Derivatives of the enzymes described herein such as His-tagged versions and others that permit easier purification can be used.

The above described mutant C5-methyltransferase enzymes can be used in a method for modifying a DNA molecule.

In particular, the above described mutant C5-methyltransferase enzymes can be utilized in part (a) step (i) and in part (b) of the method of labeling according to the first aspect of the invention. In particular, the above described mutant C5-methyltransferase enzymes can be used individually, or in combination to label DNA fragments. Part (a) step (i) and/or part (b) can be repeated for each methyltransferase, or alternatively a number of methyltransferase enzymes can be used together. Further one co-factor or several different co-factors can be used.

In this regard, where more than one mutant methyltransferase is used to label the DNA fragment, a further mutant M.HhaI C5-methyltransferase enzyme can be used in the method of the present invention. The mutant M.HhaI has an amino acid sequence which comprises the mutations at Q82 and N304 such that Q82 is replaced by a glycine, serine, threonine, asparagine, alanine or valine, and N304 is replaced by a glycine, serine, threonine, alanine or valine. In this embodiment the M.HhaI enzyme can be additionally defined as having an amino acid sequence which comprises SEQ ID No: 8 and SEQ ID No: 9, and/or having an amino acid sequence which is at least 85%, more preferably at least 90% or 95%, identical to SEQ ID No: 7. More preferably, the mutant M.HhaI enzyme has an amino acid sequence which comprises the mutations Q82A, Y254S and N304A. 
SEQ ID No: 7

MIEIKDKQLT GLRFIDLFAG LGGFRLALES CGAECVYSNE WDKYAQEVYE MNFGEKPEGD
ITQVNEKTIP DHDILCAGFP CQAFSISGKQ KGFEDSRGTL FFDIARIVRE KKPKVVFMEN
VKNFASHDNG NTLEVVKNTM NELDYSFHAK VLNALDYGIP QKRERIYMIC FRNDLNIQNF
QFPKPFELNT FVKDLLLPDS EVEHLVIDRK DLVMTNQEIE QTTPKTVRLG IVGKGGQGER
IYSTRGIAIT LSAYGGGIFA KTGGYLVNGK TRKLHPRECA RVMGYPDSYK VHPSTSQAYK
QFGNSVVINV LQYIAYNIGS SLNFKPY
 SEQ ID No: 8 GFPCXAFS where X is glycine, serine, threonine, asparagine, alanine or valine
SEQ ID No: 9 GXSVVI wherein X is glycine, serine, threonine, alanine or valine

In part (a) step (i) and part (b) of the method of labeling of unmethylated CpG dinucleotides within a DNA fragment the unmethylated cytosines are modified by incubating the fragment with the above-described mutant C5-methyltransferase enzymes with a cofactor under conditions which allow for the transfer of a part of the cofactor (optionally comprising a label) onto the unmethylated CpG dinucleotide by the enzyme to form a modified CpG dinucleotide, i.e. one in which the cytosine is modified at position 5. Suitable conditions for the activity of C5 methyltransferases are known in the art and are also applicable to the mutant C5 methyltransferases described herein.

In particular, the cofactor is an AdoMet analogue (a synthetic AdoMet), which comprises a functional group (F1), such as a primary amine, or a label in place of the reactive methyl group (CH₃). When contacted with the DNA methyltransferase enzyme in the presence of a DNA molecule the enzyme transfers a part of the AdoMet analogue, for example the side chain containing the amino group or label, from the cofactor onto a cytosine, based on the enzyme's target site in a DNA sequence, to form the modified cytosine.

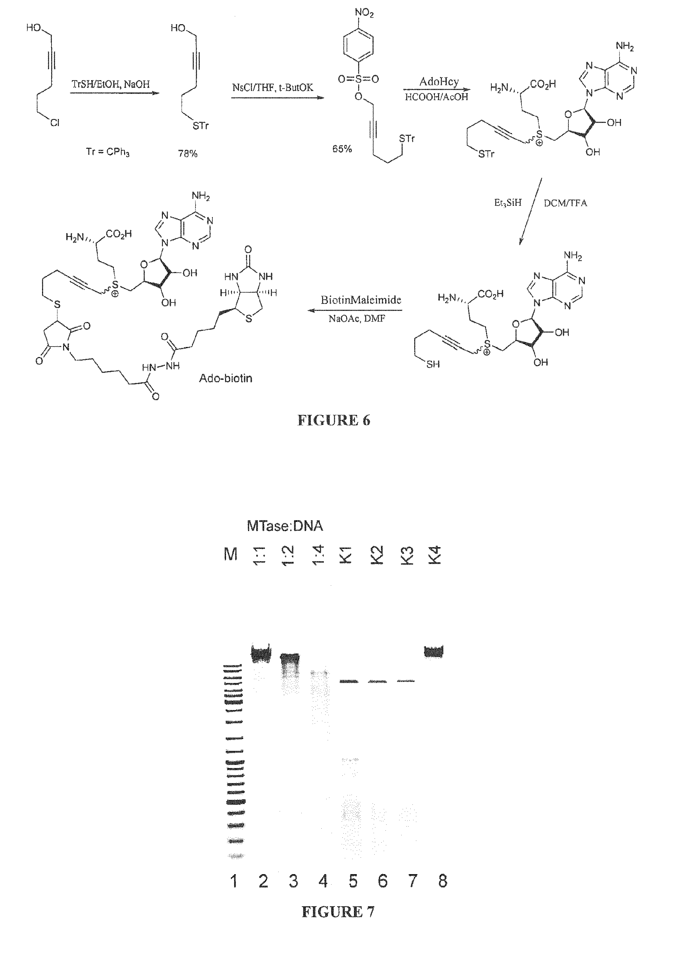

Where the part of the co-factor transferred onto the cytosine by the methyltransferase comprises a label, part (b) of the method of the invention can be performed with a co-factor as described in WO2006/108678. In one embodiment, part (b) can be performed with a co-factor comprising biotin, an example of which (Ado-biotin) is shown in Figure 6.

Where the part of the co-factor transferred onto the cytosine does not comprise a label but comprises a functional group, this functional group can be used to provide a first functional or reactive group (F1) that can be reacted in part (a) step (ii) with a compound comprising a label and a second reactive or functional group (F2). The second functional group is suitable for use with the first functional group, such that in step (ii) the first functional group reacts with the second functional group transferring the label onto the DNA sequence.

Where a functional group is transferred onto the cytosine in part (a) step (i) the cofactor is preferably a compound represented by formula (I), which is provided in a further aspect of the present invention. In particular the compound of formula (I) has the following structure: 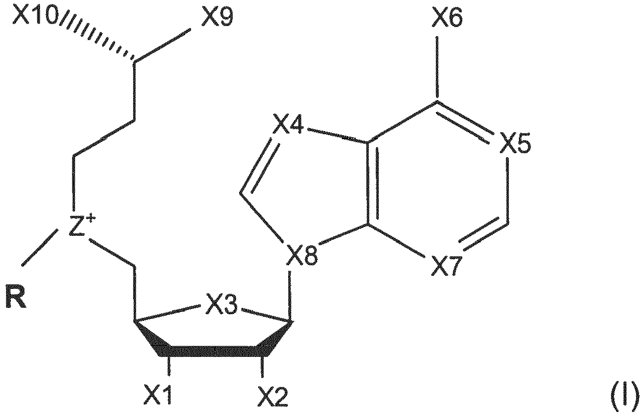 where
X1 and X2 represent -OH, -NH₂, -SH, -H or -F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH, -OCH₃, -H, -F, -Cl, -SH or -NHCH₃, and preferably is - NH₂;
X9 represents -CO₂H, -PO₃H, -H, -CHO, -CH₃, or -CH₂OH, and preferably is -CO₂H;
X10 represents -NH₂, -OH, -H, -CH₃, or -NHCH₃, and preferably is -NH₂;
X⁻ is an organic or inorganic anion selected from trifluoroacetate, formate, halide and sulfonate;
Z represents S or Se, and preferably is S;
C-bound H atoms in the adenosine moiety can be replaced by -F, -OH, -NH₂, or -CH₃, but are preferably H.

In the compound of formula (I) R comprises -CH=CH- or -C≡C- in a β-position to Z+ centre and separated therefrom by CR1R2-, where R1 and R2 are independently H or D, but are preferably H.

It has previously been demonstrated that allylic and propargylic side chains can be efficiently transferred by DNA methyltransferases with high sequence and base specificity (Lukinavi 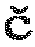 ius 2007, J. Am. Chem. Soc.). In particular, placing a double or triple bond next to the reactive carbon of AdoMet is known to be important to maintain the reaction rate. Accordingly the compound represented by formula (I) comprises a carbon-carbon double bond or a carbon-carbon triple bond in the group R next to the reactive carbon, i.e. the carbon within the group CR1R2.

R further comprises a functional group selected from an amino group, a thiol group, a 1,2-diol group, a hydrazine group, a hydroxylamine group, a 1,2-aminothiol group, an azide group, a diene group, an alkyne group (a terminal ethynyl group or a torsionally strained alkyne such as a cyclooctyne (BARAC, DIFO, DIBO, DBCO etc)), an arylhalide group, a terminal silylalkyne group, an N-hydroxysuccinimidyl ester group, a thioester group, an isothiocyanate group, an imidoester group, a maleimide group, a haloacetamide group, an aziridine group, an arylboronic acid group, an aldehyde group, a ketone group, a phosphane ester group, a dienophile group, a terminal haloalkyne group. Preferably the functional group is an amino group, a thiol group, a 1,2-diol group, a hydroxylamine group, an azide group, a diene group, a terminal alkyne group, an arylhalide group, a maleimide group, an arylboronic acid group, an alkyne group, an aldehyde group, a ketone group, or a dienophile group. Most preferably the functional group is an amino group.

Optionally, R may comprise the functional group in a protected form, such as a protected amino group, a protected thiol group, a protected 1,2-diol group, a protected hydrazino group, a protected hydroxyamino group, a protected aldehyde group, a protected ketone group, and a protected 1,2-amionthiol group.

In a preferred embodiment the functional group is a terminal functional group or a terminal protected functional group, i.e. the functional group, optionally in protected form, is at the end of R removed from the Z+ centre.

The distance in R between -CH=CH- or -C=C- in a β-position to Z+ centre and the nearest electronegative atom or group in R is based on the strength of the electronegative atom or group. It has been found that separating the double or triple bond from the nearest electronegative group or atom in R with carbon atoms can increase the stability of the cofactor in aqueous solution, i.e. the gap provides a distance suitable to block the electronegative effect of the group or atom.

An electronegative group or atom is one which, in the context of R, has a greater tendency to attract electrons towards itself than the carbon atoms involved in the double or triple bond. The electronegative group may be the functional group or may be a "connector group", i.e. be in the portion of R which links the -CH=CH- or -C=C- in a β-position to Z+ centre to the functional group. Such a connector group may be part of the main chain connecting the functional group to the -CH=CH- or -C≡C-, or may be in a side chain. The electronegative atom may be a heteroatom, such as O, N, S, Br, Se, Cl, F, and may be in the main chain or pendant from the main chain.

The required number of carbon atoms in the length between the -CH=CH- or -C=C- and the nearest electronegative group or atoms should be chosen depending on the strength of the electronegative atom or group. For groups with lower electronegativity (e.g. thiol, alkyne, diene, silylalkyne) a shorter distance such as no carbon atoms, i.e. the group is attached directly to - CH=CH- or -C≡C-, or one or two carbon units can be used. Where, however, a more electronegative group or atom is present, such an amino group, a heteroatom such as O, N, S, Br, Se, Cl or F, an azide, an n-maleimide or a hydrazide, it is preferably to have at least two or three carbon units separating the carbon involved in the double/triple bond and the electronegative group or atom.

Accordingly, in a preferred embodiment of the method of the present invention, and in the compound of the invention, the distance between -CH=CH- or -C≡C- and the nearest electronegative atom in R or the nearest electronegative group in R is at least 2 carbon atoms. By "at least two carbon atoms" is meant by a chain length of at least two carbons, e.g. -(CH)₂-, - CH=CH-, which may be branched or unbranched. Where the chain is branched the "carbon units" refer only to the carbons in the chain directly linking the -CH=CH- or -C≡C- and the nearest electronegative group or atom, and does not include any carbons that may be present in the branches/side chains. Where such branches are present it is preferably that these are C₁ to C₃ alkyl, more preferably -CH₃-. However, it is most preferred that the carbon units are -CH₂- units. Preferably in the compound of the invention, and in one embodiment of the method of the invention, the distance between -CH=CH- or -C=C- and the nearest electronegative atom or group in R is 2 or 3 carbon units.

Where the nearest electronegative group or atom is an atom it is preferred that this is selected from N, O, S, Br, Cl, F or Se.

The nearest electronegative group may be the functional group. This is a preferred embodiment for the compound of the present invention. In this embodiment R may consists essentially of - CH=CH- or -C≡C- in a β-position to Z+ centre; a functional group as indicated above, and two or three carbon units separating the -CH=CH- or -C≡C- from the functional group.

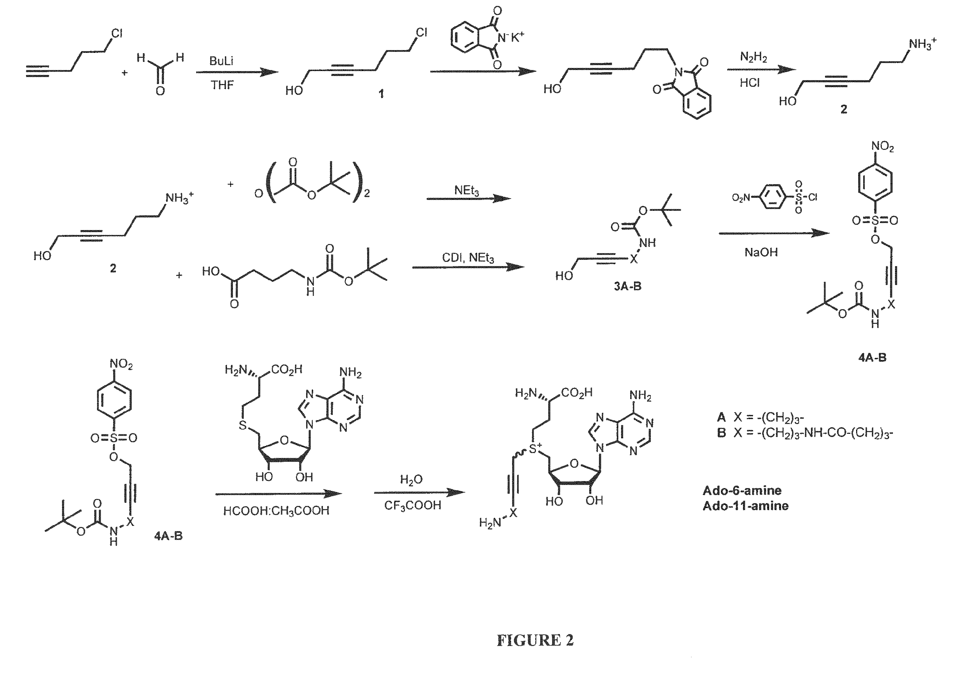

In the compound of the present invention, and in particular embodiments of the method of the invention, the distance between -C=C- or -C=C- and the functional group is no more than 7 atoms in length, i.e. the functional group and the carbon involved in the double/triple bond are separated by a chain which is no more than 7 atoms in length. More preferably, the part of R attached to the -CR1R2-CH=CH- or -CR1R2-C≡C-, has a chain which does not exceed a total of seven, more preferably six, atoms in length (including the functional group). The definition of the compound of the invention does not include Ado-11-amine, which has previously been described in Neely et al., (Chemical Science, 2010, 1,453-460) and is shown in Figure 2. This compound has a length of 8 atoms between the functional group and the carbon involved in the double/triple bond. In particular, the present inventors have found that the compounds of the present invention in which the group R is limited in length as indicated above, work particularly efficiently with the mutant enzymes of the present invention, and in particular, with the mutant of M.SssI.

In further preferred embodiments of the compound of the invention, and in preferred embodiments of the method of the invention, R comprises -C=C- in a β-position to Z+, and the functional group comprises an amino group. More preferably, in these embodiments the amino group is separated from the -C≡C- by -CR3R4-CR5R6-CR7R8- where R3 to R8 are independently H or a C₁ to C₃ alkyl. Most preferably R has the formula -CH₂C=C(CH₂)₃NH₂ (Ado-6-amine, shown in Figure 2).

As indicated above, the present inventors have surprisingly found that some mutant C5 methyltransferase enzymes work particular well with specific co-factors. Accordingly, it is preferred that where the mutant C5 methyltransferase enzymes is M.SssI as described above, a cofactor of formula I is used, having an R group comprising -C=C- in a β-position to Z+ centre, and a functional group which is an amino group. More preferably, the functional group is -NH₂- and is separated from the -C=C- by -CR3R4-CR5R6-CR7R8- where R3 to R8 are independently H or a C₁ to C₃ alkyl. Most preferably R has the formula -CH₂C≡C(CH₂)₃NH₂ (Ado-6-amine).. Further, it is preferred that where the mutant C5 methyltransferase enzymes is M.HhaI and M. HpaII, a cofactor of formula I is used, having an R group comprising -C=C- in a β-position to Z+ centre and a functional group comprising an amino group. More preferably, the functional group is separated from the -C=C- by connector group comprising -NHCO- in which the -N- atom is separated from the -C=C- by three carbon units. Most preferably R has the formula - CH₂C≡C(CH₂)₃NHCO(CH₂)₃ NH₂ (Ado-11-amine).

In view of the above, the present invention further provides the use of the compounds of the present invention in a method for modifying a target molecule, preferably DNA.

The cofactor compounds can be produced by chemical synthesis, known in the art and/or according to examples described herein. In particular, the present invention provides a method of producing the compounds described above (which comprise the group R) comprising a step of reacting an activated compound comprising R with a compound of formula IV: 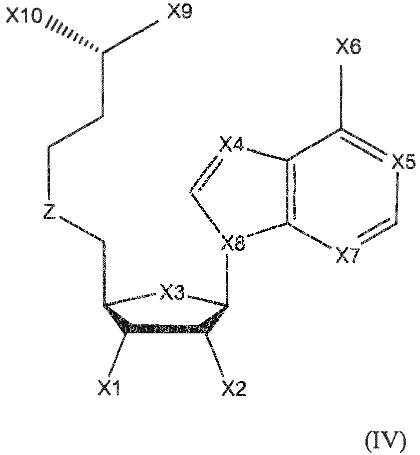 where
X1 and X2 represent -OH, -NH₂, -SH, -H or -F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH, -OCH₃, -H, -F, -Cl, -SH or -NHCH₃, and preferably is - NH₂;
X9 represents -CO₂H, -PO₃H, -H, -CHO, -CH3, or -CH₂OH, and preferably is -CO₂H;
X10 represents -NH₂, -OH, -H, -CH₃, or -NHCH₃, and preferably is -NH₂;
Z represents S or Se, and preferably is S;
C-bound H atoms in the adenosine moiety can be replaced by -F, -OH, -NH₂, or -CH₃, but are preferably H;
under conditions which allow the R group to be coupled to the Z of the compound of formula IV.

In a preferred embodiment in the method of producing a compound the activated compound comprising R is activated with an aryl sulfonate or an alkyl sulfonate containing from 1 to 3 electron-withdrawing groups. More preferably the electron-withdrawing groups are selected from nitro, nitrile, halogen, carboxyl, sulphone or sulfate.

In an additional or alternative preferred embodiment in the method of producing a compound the activated compound comprising R further comprises a protective group attached to the functional group. More preferably the protective group is *N*-BOC, 1-adamatyloxycarbonyl, trimethylsilylethyloxycarbonyl, nitrophenyloxycarbonyl, nitrophenylethyloxycarbonyl, or dimethoxynitrobenzyloxycarbonyl (DMNB).

In particular, the aspect of the invention relating to the method of producing a compound R comprises an activating group attached to CR1R2. In particular, the activated compound comprising R can comprise as the activating part aryl sulfonates (or alkylsulfonates) containing from 1 to 3 electron-withdrawing groups such as nitro, nitrile, halogen, carboxyl, sulphone, sulfate could in principle be used. Activating reagents would be corresponding arylsufonylchlorides.

Further, the activated compound comprising R preferably further comprises a protective group attached to the functional group of R. Any protective groups that is stable in formic acid and can be removed under slightly more acidic conditions are suitable such as 1-adamatyloxycarbonyl (removed with TFA) or trimethylsilylethyloxycarbonyl (removed with fluoride), etc (Greene's protective groups in organic synthesis. -4th edition/ PGM Wut and TW Greene, 2007, Wiley and Sons, Hoboken, New Jersey. p. 696-802). Also suitable are groups that are removed by light, such as nitrophenyloxycarbonyl or nitrophenylethyloxycarbonyl groups (ibid, p. 767), or dimethoxynitrobenzyloxycarbonyl (DMNB) or similar groups (J.E.T. Corrie. Dynamic Studies in Biology. Eds. M. Goeldner, R. Givens, 2005, Wiley-VCH. p.1-28). However, preferably the protective group is *N*-BOC.

Preferably where R comprises a functional group which is a primary amine, the method comprising the steps of:
i) protection of -NH₂ group and activation of -OH group in a compound represented by the formula (II) or the formula (III): 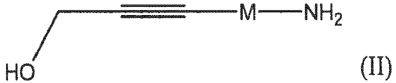 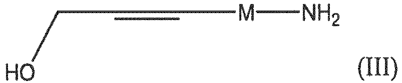 in which M is -CR3R4-CR5R6- or -CR3R4-CR5R6-CR7R8-, wherein R3 to R8 are independently H or an alkyl group.
ii) reaction of the compound produced from step (i) represented by the formula (III) with a compound represented by the formula (IV):
iii) deprotection of the protected -NH₂ group to form the compound.

Preferably, the -NH₂ group is protected by reaction with the following compound: 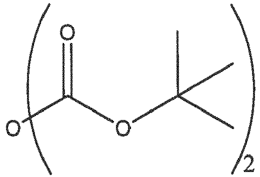 and/or the -OH group is activated by reaction with the following compound:

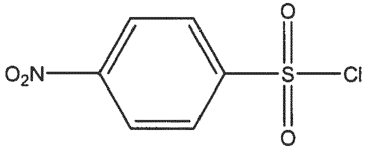

In particular, the co-factors Ado-6-amine and Ado-11-amine can be synthesized from 5-chloro-pentyne-1 via a N-BOC-protected 6-amino-2-hexyne-1-ol intermediate, whose synthesis is shown in Figure 2.

The compounds are produced as a mixture of R and S isomers as a result of chirality at the Z+. Chemical synthesis produces a mixture of both at varied ratios close to 50%. Only the S isomer is active in enzymatic reactions, so either a purified preparation enriched in the S isomer can be used (obtained by chromatographic separation) or a racemic mixture of both can be used.

As indicated above, where in the method of labeling the cytosine is not modified with a label, in part (a) step (ii) the modified cytosine residue is reacted with a compound comprising a label under conditions that allow the transfer of the label to the cytosine residue. In particular, the compound comprising the label also comprises a second functional group (F2) which reacts with the functional group (F1 - obtained from group R of formula (I)) on the modified cytosine residue, transferring the label onto the DNA fragment. Suitable groups for F2 are given below.

Suitable reactive groups for F1 and F2 are shown in Table 1. Suitable conditions for reaction between F1 and F2 are known in the art. Examples are provided herein and described in WO2006/108678.

**Table 1. Reactive functional groups F1 and F2 may comprise a variety of combinations**

| Reactive group F1 or F2 | Reactive group F 1 or F2 | Stable chemical linkage |
|---|---|---|
| Primary amine | *N*-hydroxysuccinimidyl ester | amide |
| Primary amine | thioester | amide |
| Primary amine | isothiocyanate | thioureas |
| Primary amine | imidoester | imidate |
| Primary amine | aldehyde, ketone | imine (amine after reduction) |
| Thiol | maleimide | thioether |
| Thiol | haloacetamide | thioether |
| Thiol | aziridine | thioether |
| Thiol | thiol | disulfide |
| 1,2-Diol | arylboronic acid | cyclic ester |
| Hydrazine | aldehyde, ketone | hydrazone |
| Hydroxylamine | aldehyde, ketone | oxime |
| 1,2-Aminothiol | aldehyde, ketone | thiazolidine |
| 1,2-Aminothiol | thioester | amide |
| Azide | alkyne | 1,2,3-triazole |
| Azide | phosphane ester | amide |
| Diene | dienophile | cyclohexene |
| Terminal alkyne | arylhalide | arylalkyne |
| Arylhalide | arylboronic acid | biaryl |
| Terminal silylalkyne | terminal haloalkyne | diyne |

Particularly preferred functional groups are primary amine, thiol, 1,2-Diol, hydroxylamine, azide, diene, terminal alkyne, arylhalide, aldehyde, ketone, maleimide, alkyne, dienophile, arylhalide and arylboronic acid.

Optionally, the functional group in a protected form, such as a protected amino group, a protected thiol group, a protected 1,2-diol group, a protected hydrazino group, a protected hydroxyamino group, a protected aldehyde group, a protected ketone group, and a protected 1,2-amionthiol group.

As such, the reactive F1 group may be first transferred in a protected form as a derivative that is converted to an active functional form in a separate step. For example, thiols may be transferred with acetyl protecting group (protected F1= -S-COCH₃) which can be readily removed to yield thiol (F1 = -SH) by treatment of modified DNA with 20% ammonia, or transferred 1,2-diol can be converted to aldehyde by oxidation with sodium periodate.

Suitable labels for use in the present invention are known in the art. In particular, the labels are those which can be used in enrichment procedures, such as affinity tags. Accordingly, the label can be selected from c-myc-tag, HA-tag, digoxygenin, flag-tag, dinitrophenol, His tag, biotin, strep-tag, glutathione, nickel-nitrilotriacetic acid (NTA), maltose, oligonucleotide primer, DNA or RNA aptamer. In a preferred embodiment the label is biotin, which enables the use of enrichment procedures involving the binding partner streptavidin. Accordingly, the compound comprising the label for use in step (ii) can be Biotin-SS-NHS (commercially available from Sigma, Cat.No.B453!).

The present invention further provides a method of genomic DNA methylation profiling using the method of labeling of the invention described above.

In particular, in a further aspect the present invention provides a method for analysing unmethylated CpG dinucleotides within one or more DNA molecules, comprising the steps of:
(a) providing fragments of the DNA molecules;
(b) labeling the unmethylated CpG dinucleotides according to the methods described above to produce labeled DNA fragments;
(c) enriching the labeled DNA fragments;
(d) amplifying the labeled DNA fragments; and
(e) analyzing the amplified DNA fragments to determine the methylation status of the CpG dinucleotides.

In a preferred embodiment the one or more DNA molecules are genomic DNA.

The DNA fragments or oligonucleotide segments are not especially limited and are simply subsequences or sections of nucleic acid. The segments may be formed by mechanical methods or by enzymatic or chemical digestion of the nucleic acid. The segments are preferably formed by DNA shearing. The oligonucleotide segments are usually double stranded. Preferably they are from 50 to 500 bp in length, more preferably they are from 50 to 300 bp in length.

The method for analysing may further comprise a step after step (a) but prior to step (d) of ligating an adaptor to the 5' and the 3' end of each fragment or segment, wherein the adaptor comprises a nucleic acid sequence capable of hybridizing with a primer for a polymerase chain reaction. Typically, the segments formed are blunt-ended with T4 DNA Polymerase or the other suitable enzyme, and the adaptor nucleic acid sequence is ligated to each of the 5' and 3' blunt ends. Alternatively, the segments have sticky ends, and the adaptor nucleic acid sequence is ligated to the sticky ends. The skilled person will be well aware of suitable methods for ligating adaptor sequences to nucleic acid segments. Suitable ligation enzymes include T4 DNA Ligase.

Enrichment of the labeled DNA fragments in step (c) is completed utilizing the label and generally comprises affinity purification. Such a step usually involves a ligand immobilized on a solid phase (such as the surface of a bead). The labeled DNA fragments are contacted with the ligand and the label binds to the ligand, enabling the labeled DNA fragments to be separated from the unlabeled DNA fragments. In a preferred embodiment the label is biotin and step (c) comprises contacting the labeled fragments with streptavidin-coated beads under conditions which allow the binding of the biotin to the streptavidin, removal of the unlabeled DNA fragments and recovery of the captured labeled DNA from the beads.

Recovery of bound DNA can be achieved by a) denaturation of streptavidin with suitable reagents, b) competing binding of free biotin or c) selective chemical or enzymatic cleavage of the connecting linker that contains a specific chemical linkage/bond. The latter approach has an advantage that the DNA fragments contain a shorter covalent side chain attached (no biotin moiety) which is beneficial for downstream applications such as PCR amplification (where larger extension can interfere with -slow down or block- polymerase action). Preferably, a disulphide linkage -S-S- is cleaved under mild conditions with reducing agents such as DTT or 2-mercaptoethanol. Other possibilities are: a cis-diol moiety -CH(OH)-CH(OH)- which can be cleaved by treatment with sodium periodate; a selenoether linkage -Se- which can be cleaved by treating with an oxidant (sodium periodate or hydrogen peroxyde) to give selenoxide, which can subsequently undergo elimination with the cleavage of a Se-C bond (Wirth, T. (2000) Angew. Chem. Int. Ed. 39, 3740-3749; Gieselman et al. (2002) ChemBioChem 3, 709-716).

The recovered labeled fragments can be amplified using PCR methods known in the art.

In step (e) the amplified DNA fragments can be analysed also using methods known in the art. In particular, step (e) may comprise microarray analysis and/or it may comprise next generation sequencing of the enriched nucleic acid fragments. Methods of sequencing nucleic acid fragments are well known to a person skilled in this art.

In a particularly preferred embodiment the DNA molecules are labeled using the mutant M.SssI, mutant M. HpaII and mutant M.HhaI enzymes described above in combination.

In a further aspect the present invention provides a kit comprising the compound of the invention an a methyltransferase enzyme. In particular, these kits can be used in a method for labeling target molecules, preferably DNA. The kit comprises the compound of the invention as described above is a suitable container, in combination with a methyltransferase in a suitable container. The methyltransferase is not particularly limited but is one which normally uses S-adenosyl L-methionine (SAM or AdoMet) as a cofactor. Preferably the methyltransferase enzyme is a DNA methyltransferase, and still further may be is a CpG C-5 methyltransferase enzyme.

More preferably the CpG C-5 methyltransferase enzyme is an enzyme according to the present invention as described above, or is M.HhaI, wherein the M.HhaI comprises mutations at Q82 and N304, wherein Q82 is replaced by a glycine, serine, threonine, asparagine, alanine or valine, and N304 is replaced by a glycine, serine, threonine, alanine or valine. Still more preferably the M.HhaI further comprises the mutation Y254S, and preferably also comprises the mutations Q82A and N304A.

In a further aspect the present invention provides a kit comprising at least two methyltransferase enzymes according to the present invention as described above. In particular, this kit can be used in a method for labeling DNA. The kit comprises more than one of the above described mutant C5 methyltransferase enzymes of the invention in a suitable container.

In a still further aspects the present invention provides a complex of a compound according to any formula (I) and a methyltransferase with is capable of using S-adenosyl-L-methionine (SAM or AdoMet) as a cofactor. Preferably the compound is a compound according to the present invention as described above. Preferably the methyltransferase is one which is capable of transferring or which normally transfers the methyl residue of AdoMet onto a nucleic acid molecule, a polypeptide, a carbohydrate or a small molecule, such as a phospholipid, an amino acid, a hormone, a nucleotide, a nucleoside or a derivative thereof. More preferably in the complex the methyltransferase is a C5 DNA methyltransferase, and most preferably the enzyme is one of the mutant C5 DNA methyltransferases described above.

Still further the present invention provides a nucleic acid molecule modified with an R group from a compound of formula (I) as defined above. Specifically, the nucleic acid molecule comprises at least one residue in which a cytosine base is derivatised at position 5 with a group R, wherein R comprises -CR1R2-CH=CH- or -CR1R2-C=C-, where R1 and R2 are independently H or D, and wherein R further comprises a functional group selected from an amino group, a thiol group, a 1,2-diol group, a hydrazine group, a hydroxylamine group, a 1,2-aminothiol group, an azide group, a diene group, an alkyne group, an arylhalide group, a terminal silylalkyne group, an N-hydroxysuccinimidyl ester group, a thioester group, an isothiocyanate group, an imidoester group, a maleimide group, a haloacetamide group, an aziridine group, an arylboronic acid group, an aldehyde group, a ketone group, a phosphane ester group, a dienophile group, a terminal haloalkyne group,
wherein the distance between the - CH=CH- or -C=C- and the functional group is no more than 7 atoms in length,
and wherein the distance between -CH=CH- or -C=C- and the nearest electronegative atom or group in R is at least 2 carbon atoms.

Preferred features for R in the modified nucleic acid molecule are the same as those described above in relation to the compound of the present invention.

In particular, preferably the nearest electronegative atom is selected from N, O, S, Br, Cl, F or Se.

Preferably the functional group is a terminal functional group or a terminal protected functional group.

Preferably the nearest electronegative group is the functional group.

Preferably the -CH=CH- or -C=C- is separated from the functional group by two or three carbon units, and more preferably the -CH=CH- or -C≡C- is separated from the functional group by - CR3R4-CR5R6- or -CR3R4-CR5R6-CR7R8-, wherein R3 to R8 are independently H or a C₁ - C₃ alkyl.

Preferably the functional group is an amino group, a thiol group, a 1,2-diol group, a hydroxylamine group, an azide group, a diene group, a terminal alkyne group, an arylhalide group, a maleimide group, an arylboronic acid group, an aldehyde group, a ketone group or a dienophile group, more preferably the functional group is an amino group, still more preferably R is 6-aminohexyn-2-yl.

The nucleic acid molecule may be DNA or RNA, but is preferably DNA. Most preferably, the nucleic acid molecule comprises at least one modified cytosine residue which is 5-(6-aminohexyn-2-yl)-2'-deoxycytidine.

The invention is further illustrated by the following examples:

### Example 1: Design and chemical synthesis of AdoMet analogs

Studies of the stability of the previously described cofactor (Ado-9-amine, Lukinavicius et al. 2007) containing the butyn-2-yl moiety showed its short halflife (7 minutes) in reaction buffers due to addition of a water molecule to the triple bond. We thus replaced the butynyl shuttle moiety with a hexyn-2-yl moiety such that the separation between the triple bond and the polar amido group is increased from 1 to 3 carbon units. Two synthesized cofactors, Ado-6-amine and Ado-11-amine co-factors, with the overall side chain length of 6 and 11 units, respectively, showed much higher halflifes (~ 2 h) in reaction buffers.

Figure 2 shows the structure and general synthetic route to Ado-6-amine and Ado-11-amine cofactors. In particular, synthesis of the new cofactors included a N-BOC-protected 6-amino-2-hexyne-1-ol intermediate, which was obtained from 5-chloro-pentyne-1 in three synthetic steps as shown in Figure 2.

Chemical synthesis of Ado-6-amine and Ado-11-amine cofactors according to steps shown in Figure 2 is as follows:
6-Chlorohex-2-yn-1-ol (**1**)
   Butyllithium (24 mmol, 1 equiv.) was added to 24 mmol (2.5 ml; 1 equiv.) of 5-chloropent-1-yne in 30 ml anhydrous THF under argon, and the mixture was stirred for 30 min at -70°C. After addition of 26 mmol (0.84 g; 1.1 equiv.) of paraformaldehyde, and stirring was continued for 30 min at -70°C and then for 1 h at room temperature. The reaction was quenched with 30 ml of cold water, the aqueous phase was extracted twice with diethyl ether and the combined organic phase was dryed with anhydrous MgSO₄. The solvent was removed under reduced pressure to give 6-chlorohex-2-yn-1-ol (1).
   ¹H-NMR (300 MHz, CDCl₃): δ = 1,95 (quint, ³J = 6,6 Hz, 2H, CH₂), 2,41 (tt, ³J = 6,7 Hz, ⁵J = 2,2 Hz, 2H, CH₂), 2,77 (br. s., 1H, OH), 3,64 (t, ³J = 6,4 Hz, 2H, CH₂), 4,23 (t, ⁵J = 2,2 Hz, 2H, CH₂). ¹³C-NMR (75 MHz, CDCl₃): δ = 15,49; 25,78; 38,80; 49,91; 79,58; 84,62
6-Aminohex-2-yn-1-ol (**2**)
   6-Chlorohex-2-yn-1-ol (**1**) (2.00 g, 1 equiv.) was added to a solution (30 ml) of potassium phtalimide (3.15 g, 1.1 equiv.) in DMF and heated at 80°C for 1 h. Solvent was removed by evaporation under reduced pressure and liquid 6-phtalimidohex-2-yn-1-ol was dissolved in methanol (150 ml). Hydrazine hydrate (3.46 ml, 2 equiv.) was added and the reaction was heated with reflux for 2 h and after cooling to room temperature the solvent was removed under reduced pressure. Water, ethanol and conc. hydrochloric acid were added, mixture was heated with reflux for 20 min and the precipitate removed by filtration. The filtrate was concentrated under reduced pressure.
   6-Aminohex-2-yn-1-ol hydrochloride (**2**), yield 70%. ¹H-NR4R (300 MHz, CDCl₃): δ = 1.88 (quint, ³J = 7.5 Hz, 2H, CH₂), 2.39 (tt, ³J = 6.9 Hz, ⁵J = 2.2 Hz, 2H, CH₂), 3.13 (t, ³J = 7.5 Hz, 2H, CH₂), 4.22 (t, ⁵J = 2.2 Hz, 2H, CH₂); ¹³C-NMR (75 MHz, CDCl₃): δ = 15.49; 25.78; 38.80; 49.91; 79.58; 84.62.
6-(BOC-amino)hex-2-yn-1-ol (3A)
   The protection of primary amino group with a tert.-Butoxycarbonyl (Boc) group was performed according to Greene (Greene, T. W. and P. G. M. Wuts (1999). Protective groups in organic synthesis, 3rd edition, John Wiley & Sons, New York , 518-525).
   6-(tert.-Butoxycarbonylamino)hex-2-yn-1-ol (3A), yield 80 %. ¹H-NMR (300 MHz, CDCl₃): δ = 1.35 (s, 9H, CH₃); 1.60 (quint, ³J = 6.9 Hz, 2H, CH₂), 2.18 (tt, ³J = 6.9 Hz, ⁵J = 2.0) Hz, 2H, CH₂), 3.13 (q, ³J = 6.4 Hz, 2H, CH₂), 3.48 (br. s., 1H, OH), 4.14 (br. s., 2H, CH₂), 4.90 (br. s., 1H, NH); ¹³C-NMR (75 MHz, CDCl₃): δ = 16.39; 28.65; 28.86; 39.76; 51.05; 79.56; 79.82; 84.89; 123.53; 156.46.
6-(BOC-aminobutanamido)hex-2-yn-1-ol (**3B**)
   4-[(tert.-butoxycarbonyl)amino]butanoic acid (1 equiv., 5 g, prepared in analogy to (Greene et al., 1999) was dissolved in anhydrous tetrahydrofuran (20 ml), carbonyldiimidazole (CDI) (1.1 equiv., 4.56 g) was added, and the resulting clear solution was stirred at room temperature for 2 h. Then, 6-aminohex-2-yn-1-ol hydrochloride (**2**) (1 equiv.) and trietylamine (2 equiv.) were added and stirring was continued at room temperature for 2 h. The solvent was removed under reduced pressure and the crude product was purified by column chromatography (silica gel). Product containing fractions were pooled and solvent was removed under reduced pressure.
   6-[(tert.-Butoxycarbonylamino)butanamido]hex-2-yn-1-ol (**3B**), yield 60%. ¹H-NMR (300 MHz, CDCl₃): δ = 1.45 (s, 9H, CH₃), 1.69-1.87 (m, 4H, CH₂), 3.16 (t, ³J = 6.5 Hz, 2H, CH₂), 3.39 (q, ³J = 6.5, 2H, CH₂), 4.24 (t, ⁵J = 2.2 Hz, 2H, CH₂), 5.06 (br. s, 1H, NH), 6.81 (br. s, 1H, NH); ¹³C-NMR (75 MHz, CDCl₃): δ = 16.74; 26.65; 28.21; 28.66; 33.89; 39.01; 40.14; 51.12; 79.73; 80.08; 84.99; 159.93; 173.41.

### Activation of alcohols by sulfonylation

4-Nitrobenzenesulfonyl chloride 1.1 equiv., 0.90 g) and sodium hydroxide (5 equiv., 0.74 g) were added to a solution of protected aminoalcohol (**3A-B**)(1 equiv.) in methylene chloride (15 ml) at 0°C. After stirring the reaction mixture for 3 h at room temperature sodium hydroxide was filtered, the reaction was quenched with 20 ml of cold water, extracted with methylene chloride and the combined organic layers dried over sodium sulfate. The sample was passed through a glass filter and concentrated to a yellowish solid.

6-(tert.-Butoxycarbonylamino)hex-2-ynyl-4-nitrobenzenesulfonate (**4A**), yield 50%. ¹H-NMR (300 MHz, CDCl₃): δ = 1.41 (s, 9H, CH₃); 1.53 (quint, ³J = 7.0 Hz, 2H, CH₂), 2.09 (tt, ³J = 7.0 Hz, ⁵J = 2.2 Hz, 2H, CH₂), 3.06 (q, ³J = 6.7 Hz, 2H, CH₂), 4.57 (br. s., 1H, NH), 4.80 (t, ³J = 2.2 Hz, 2H, CH₂), 8.10-8.14 (m, 2H, arom. H), 8.36-8.41 (m, 2H, arom. H); ¹³C-NMR (75 MHz, CDCl₃): δ = 16.35; 28.56; 28.63; 39.72; 60.03; 72.23; 79.61; 79.65; 90.76; 124.61; 129.74; 142.55; 151.05; 156.14.

6-[4-(*tert*.-Butoxycarbonylamino)butanamido]hex-2-ynyl-4-nitrobenzenesulfonate (**4B**), yield 50%. ¹H-NMR (300 MHz, CDCl₃): δ = 1.37 (s, 9H, CH₃); 1.55 (quint, ³J = 7.0 Hz, 2H, CH₂), 1.74 (quint, ³J = 6.8 Hz, 2H, CH₂), 2.09 (tt, ³J = 7.1 Hz, ⁵J = 2.2 Hz, 2H, CH₂), 2.19 (t, ³J = 7.1 Hz, 2H, CH₂), 3.03-3.21 (m, 4H, CH₂), 4.77 (t, ⁵J = 2.2 Hz, 2H, CH₂), 5.13 (br. s., 1H, NH), 6.87 (br. s., 1H, NH), 8.07-8.13 (m, 2H, arom. H), 8.33-8.40 (m, 2H, arom. H); ¹³C-NMR (75 MHz, CDCl₃): δ = 16.48; 26.59; 27.95; 28.59; 33.57; 38.75; 39.98; 60.11; 72.23; 79.48; 90.72; 124.65; 129.69; 142.36; 151.04; 156.87; 173.45.

### S-Alkylation of S-adenosyl-L-homocysteine

4-nitrobenzenesulfonyl ester (**4A-B**, 4-30 equivalents) was slowly added to S-adenosyl-L-homocysteine (1 equiv., 10-20 mg) in a 1:1 mixture of formic acid and acetic acid (0.5-1.0 ml) at 0°C. The solutions were allowed to warm up to room temperature and incubated with shaking. After a specified time (2-8 h) the reaction was quenched with water. The aqueous phase was extracted with an equal volume of diethyl ether and was concentrated in a rotary evaporator.

Deprotection of amino group was performed by adding two volumes of CF₃COOH to aqueous solution of BOC-protected AdoMet analogue and incubating for 1h at room temperature. Excess 4-nitrobenzenesulfonate was removed by passing solution through a Dowex-1 anion exchanger column. If necessary, purification of AdoMet analogs was performed by preparative reversed-phase HPLC eluting with a linear gradient of two solvents: A (20 mM HCOONH₄) and B (80% methanol). Enriched fractions were pooled and lyophilized.

**Ado-6-amine**, yield 50%. ¹H NMR (300 MHz, D₂O): δ = 1.60-1.66 (m, 1H, H5"_{R}), 1.72-1.77 (m, 1H, H5"_{S}), 1.97-2.22 (m, 3H, H4"_{R}, Hβ_{S/R}), 2.29 (t, ³J = 7.0 Hz, 1H, H4"_{S}) 2.83 (t, ³J = 7.9 Hz, 1H, H6"_{R}), 2.92 (t, ³J = 7.7 Hz, 1H, H6"_{S}), 3.30-3.75 (m, 4H, Hγ_{S/R}, Hα_{S/R}, H5'_{R}), 3.80-3.86 (m, 1H, H5'_{S}), 4.12-4.25 (m, 2H, H1"_{R/S}), 4.37-4.47 (m, 1H, H4'_{S/R}), 4.63 (quint, ³J = 5.9 Hz, 1H, H3'_{S/R}), 4.78-4.84 (m, 1H, H2'_{S/R}), 5.96 (d, ³J = 3.8 Hz, 0.5H, H1'_{S}) 5.99 (d, ³J = 2.8 Hz, 0.5H, H1'_{R}), 8.12-8.16 (m, 2H, arom. H_{S/R}).

High resolution ESI-MS analysis (Agilent 6520 Q-TOF): found m/z = 480.2020; calculated for [C₂₀H₃₀N₇O₅S]⁺ = 480.2024.

**Ado-11-amine**, yield 40%. ¹H NMR (300 MHz, D₂O): δ = 1.49 (quint, 1H, X₁₀), 1.65 (quint, 3H, H5"), 1.82-1.92 (m, 6H, H10", X₅), 2.08 (q, 1.2H, X₉), 2.20-2.35 (m, 10H, Hβ, H9", H4", X₄), 2.50 (t, 1.5H, X₆), 2.93-3.00 (m, 5.6H, H11"), 3.06 (t, 1H, X₁₁), 3.14 (t, 1H, H6"_{R}), 3.22 (t, 1H, H6"_{S}), 3.42-3.64 (m, 2.5 H, H5'_{R}, Hγ), 3.75-3.80 (m, 1H, Hα_{R/S}), 3.93-3.94 (m, 0.5H, H5'_{S}), 4.29 (br. s, 1H, H1"_{R}), 4.32 (br.s, 1H, H1"_{S}), 4.48-4.55 (m, 1H, H4'), 4.62 (t, 1H, H3'), 4.68 (t, 1.8H, X₁), 4.87-4.92 (m, 1H, H2'), 6.03-6.06 (m, 1H, H1'_{R/S}) 8.20-8.23 (m, 2H, arom. H).

Note: X signals derive from traces of 6-(4-aminobutanamido)hex-2-yn-1-ol.

### Example 2A: Selected mutants of M.HhaI, M.HpaII and M.SssI methyltransferases are capable of coupling sidechains from the cofactors Ado-6-amine and Ado-11-amine to DNA.

Our approach is based on exploiting the following three DNA methylation enzymes: M.HhaI (GCGC), M.HpaII (CCGG) and M.SssI (CG). It was also shown that engineering of the cofactor pocket of M.HhaI by conversion of certain conserved residues (Q82 and N304 in conserved motifs IV and X, respectively) to alanine leads to a significant improvement of the transalkylation activity with synthetic AdoMet analogs (Dalhoff et al., Nat Protoc. 2006;1, 1879-86, Lukinavicius et al. J. Am. Chem. Soc. 2007, 129, 2758-2759; Nelly et al., Chem. Sci. 2010, 1, 453-60).

The Y254S mutation was introduced into the original enzyme as well as into the subsequent engineered versions. We found that indeed the Y254S mutation is beneficial for the transalkylation activity and permits for lower concentrations of the cofactor analogs in the labeling reactions. Therefore, the triple Q82/Y254S/N304A mutant is now preferentially used M.HhaI variant for DNA labeling at GCGC sites.

The other two MTases, M.HpaII and M.SssI, were subcloned as His6-tagged variants, and the purification procedures for obtaining AdoMet-free enzymes were established. In the second step, appropriate changes were produced, by site-directed mutagenesis, in the HpaII (Q104A/N335A) and SssI (Q142A/N370A) MTases, and the double-alanine mutants were obtained in a similar fashion. The engineered version showed a surprisingly dramatic increase (~2 orders of magnitude) in transalkylation activity with synthetic AdoMet analogs as compared to the original His6 tagged variant for both MTases, as shown in Figures 3 and 4.

Inspired by the enhanced performance of the M.HhaI triple mutant, we attempted to further improve the efficiency of M.HpaII (Q104A/N335A) by introducing an additional alanine mutation at positions Val269, Ile284 and Ile293. Based on sequence alignments (e.g. as described in Vilkaitis et al., J. Biol. Chem. 2000, 275, 38722-38730) or on a 3D model of the HpaII methylase that was generated by an on-line automatic modeling server (Schwede et al, (2003) Nucleic Acids Res., 31,3381-85), these positions were selected for mutation as large non-charged amino acids in the vicinity of the cofactor pocket in the variable region of the C5-Mtases located between conserved motifs VIII and IX. However, the catalytic transfer of extended groups from Ado-11-amine cofactor proved weaker than that of the original double mutant and decreases in the order Q104A/N335A > Q104A/N335A/1284A >> Q104A/N335A/V269A > Q104A/N335A/I293A.

Figure 3 shows enzymatic alkylation of 1343 bp DNA fragment having 18 SssI target sites by SssI-His6 Q142A/N370A mutant with AdoMet cofactor analog Ado-6-amine. The alkylation efficiency of one SssI target site was analysed by restriction protection assay with Hin6I restriction endonuclease (target site GCGC). 1343 bp DNA fragment was treated with corresponding amount (indicated above the gel) of SssI-His6 Q142A/N370A mutant in the reaction buffer (10 mM Tris-HCl (pH7.5), 50 mM NaCl, 0.1 mg/ml BSA) supplemented with 10 mM MgCl₂ or without MgCl₂ in the presence of 40 µM Ado-6-amine for 2 hours at 37°C. After thermal inactivation of enzyme for 15 min at 80°C, Tango^{™} buffer (Fermentas) and 5u Hin6I restriction endonuclease were added to reaction mixture and it was further incubated for 3 hours at 37°C. The completion of DNA modification is described as an amount of DNA which remains protected from Hin6I-cleavage. The analysis demonstrates that alkylation is more efficient in the reaction buffer without MgCl₂: ~70% of SssI target site is protected from cleavage in comparison to ~30% in the presence of MgCl₂ (lane 2 and 8, MTase:DNA = 2:1).

Figure 4 shows the transalkylation activity of SssI-His6 Q142A/N370A mutant in the reaction buffer without magnesium ions in the presence of increasing amounts of AdoMet cofactor analog Ado-11-amine (20-320 µM). The analysis was done as described above. ~50% of SssI target site remains intact when Ado-11-amine cofactor concentration is in the range of 160-320 µM.

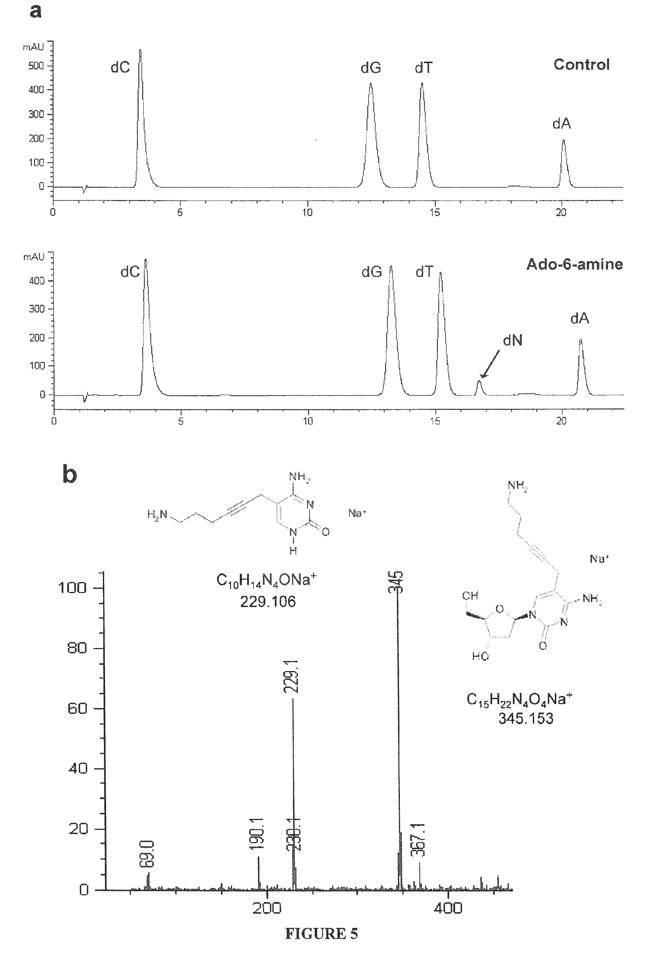

Figure 5 shows composition analysis of DNA transalkylated with M.SssI (His₆ Q142A/N370A mutant) with cofactor Ado-6-amine. Duplex oligonucleotide (10 uM, 5'-GCATTACGCGCCAGGTCGTTTCGT-3' (SEQ ID No: 32)/3'-GTAATGCGCGGTCCAGCAAAGCAT-5' (SEQ ID NO: 33)) was incubated in M.SssI buffer (10 mM Tris-HCl pH 7.6 , 50 mM NaCl, 0.2 mg/ml BSA) with 2.8 µM M.SssI and 80 µM cofactor for 2 h at 37°C. M.SssI-modified DNA samples were combined with Nuclease PI buffer (10 mM Tris-HCl, 10 mM magnesium chloride, 1 mM Zinc acetate, pH 7.5) containing nuclease PI (1.5 u) and calf intestine alkaline phosphatase (30 u) and then incubated at 42°C for 4 h. For nucleoside analysis by reversed-phase HPLC-coupled ESI-MS (Hewlett-Packard 1100), samples were loaded onto a reversed-phase HPLC column (Discovery HS C18, Supelco) and eluted with a gradient of methanol (0% for 3 min, followed by linear gradients to 20% in 15 min and to 80% in 2 min, 80% for 5 min.) in ammonium formate buffer (20 mM, pH 3.5) at a flow rate of 0.3 mL/min and at 30°C. Post-column equal co-flow of 96% methanol, 4% formic acid and 1 mM sodium formate was used for the MS detection of modified nucleosides and its derivatives in the 50-500 m/z range in positive ion mode.
a) UV trace of HPLC analysis of nucleosides formed after enzymatic hydrolysis of transalkylated DNA. dA, dC, dG and dT stands for 2'-deoxyadenosine, 2'-deoxycytidine, 2'-deoxyguanosine and thymidine respectively. Control experiment was performed without cofactor. b) ESI-MS analysis of modified nucleoside. dN denotes deoxynucleoside; B - nucleobase. HPLC analysis shows appearance of a modified nucleoside dN at 16.7 min whose molecular mass matches that of the expected 5-(6-aminohexyn-2-yl)-2'-deoxycytidine (calculated for C₁₅H₂₂N₄O₄Na M/Z = 345.153; found 345.1).

### Example 2B: Mutant of M.HhaI methyltransferases is capable of coupling a sidechain from a cofactor comprising biotin to DNA.

Figure 6 shows the synthesis of Ado-biotin cofactor.
6-Chlorohex-2-yn-1-ol was treated with triphenylmethylmercaptane (tritylmercaptane, TrSH) and then with 4-nitrophenylsulfonyl chloride (NsCl) to give S-protected-O-activated 6-mercaptohex-2-yn-1-ol. The latter is used to alkylate S-adenosylhomocyesteine (AdoHcy) as described (Lukinavicius 2007). After removal of the trityl protecting group by treatment with triethylsilane and coupling with BiotinMaleimide (N-biotinoyl-N'-(6-maleimidohexanoyl)hydrazide, Sigma B1267), racemic Ado-biotin cofactor was obtained. HRMS analysis: calculated for C₄₀H₅₈N₁₁O₁₀S₃⁺ M/Z= 948.3525; found: 948.3520

Figure 7 shows the enzymatic activity of M.HhaI with cofactor Ado-biotin.
Bacteriophage lambda DNA was treated with Ado-biotin cofactor (290 µM) in the presence of M.HhaI (variant Q82A/Y254S/N304A) for 2 h at 37C, and then modified DNA was treated with R.Hin6I and analyzed by agarose gel electrophoresis. Lane 1 Molecular mass standard GeneRuler™ DNA Ladder Mix; Lanes 2--4, DNA + cofactor + M.HhaI + R.Hin6I, molar ratios of M.HhaI to GCGC target sites (MTase:DNA) are indicated above the photograph; Lane 5-control 1, DNA + cofactor + R.Hin6I; Lane 6- control 2, DNA + R.Hin6I; Lane 7- control 3, DNA + M.SssI + R.Hin6I; Lane 8, control 4, untreated DNA. Lanes 4, 3 and 2 show increasing protection of lambda DNA against fragmentation with R.Hin6I restriction endonuclease due to M.HhaI-directed transfer of biotin containing groups from cofactor Ado-biotin onto the GCGC target sites.

### Example 3. Labeling and enrichment of unmethylated CG sites in human genomic DNA. DNA fragmentation, mTAG labeling, affinity binding and recovery procedures.

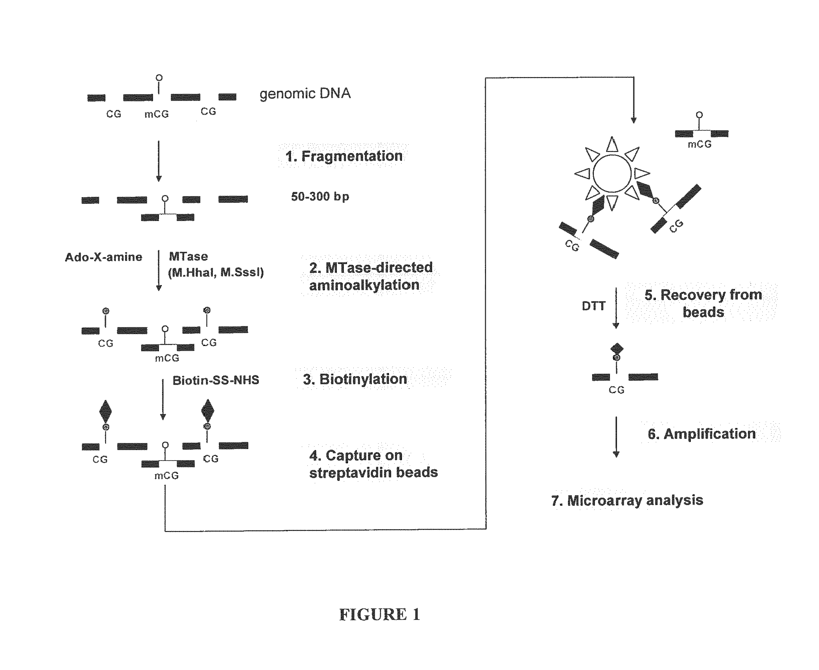

The ability of the above-described synthetic co-factors and mutant enzymes to enable successful profiling of genomic DNA methylation patterns was tested using the the analytical procedure illustrated in Figure 1. In particular, the procedure involved the following steps:
1)Shearing of genomic DNA to fragments of 50-300 bp.
2)MTase-directed functionalization/labeling of unmethylated CG dinucleotides.
3)Appending biotin reporters at the attached amino groups.
4)Affinity capture of biotin-labeled fragments on streptavidin-coated beads.
5)Recovery of the captured DNA.
6)PCR amplification of the recovered fraction for microarray analysis.
7)Microarray analysis.

Below, each step of the technology is described in detail.

### 1) Shearing of genomic DNA to fragments of 50-300 bp.

Fragmentation of genomic DNA is carried out by sonication; the average fragment size is selected depending on the expected mTAG labeling density with particular MTases (typically 100-300 bp for M.HhaI).

100 µl genomic DNA solution of human brain in 1x T4 DNA Polymerase buffer (Fermentas) at 50 ng/µl concentration is sonicated on Bioruptor UCD-200 to obtain 70-300 bp DNA fragments with the peak maximum at 150 bp. Sonication conditions are set as follows:
Pre-cool the water bath with crushed ice for 30 min. Then fill the tank with cold water (4°C), supplemented with 0.5 cm crushed ice. Bioruptor power settings are on position "High" with sonication cycling - 30 seconds "ON", 30 seconds "OFF". Sonicate for 15 min. Temperature of the water bath at the end of sonication procedure should be around 10°C. Change the water in the bath and add crushed ice as above. The temperature in the water bath can be maintained either by manual or automatic temperature control. Repeat sonication for another 8 cycles (sonication total time: 2 hours 15 min). After sonication, 2-3 µl of the DNA is analysed on an agarose gel. The optimal size of DNA fragments is 70-300 bp with a peak maximum at 150 bp.

In the next step, genomic DNA fragments are blunt-ended with T4 DNA Polymerase: 95 µl of sonicated DNA from the previous step is mixed with 5 µl of dNTP solution (0.1 mM final concentration) and 1 µl (5 u) T4 DNA Polymerase (Fermentas). The reaction is performed at 11°C for 20 min, and then stopped by heating at 75°C for 10 min. DNA is purified using QIAquick Nucleotide Removal columns with 10 V of PN Solution (Qiagen). The DNA samples are eluted of the column with EB buffer (10 mM Tris-HCl, pH 8.5).

### 2) MTase-directed functionalization/labeling of unmethylated CpG dinucleotides.

To monitor the efficiency and specificity of the labeling of unmethylated and methylated fragments throughout various step of the analytical sequence we have designed two reference systems, both consisting of a pair of 200 bp fragments (specific and nonspecific) that can be added to genomic DNA samples as internal probes.

For controlling of the labeling efficiency of HhaI and HpaII MTases, the control system was prepared from pBR322, below referred to as Control-H reference system. The specific DNA fragment of Control-H contains a single HhaI and HpaII target site, whereas no above-mentioned sites are in the nonspecific DNA fragment. Both DNA probes were prepared by PCR amplification of pBR322 DNA template with two sets of primers: I (SEQ ID NO:10) (5'-gtcctggccacgggtgc-3') and II (SEQ ID NO: 11) (5'-tccgcgtttccagactttac-3') for the specific probe, and III (SEQ ID NO:12) (5'-gtcgttcggctgcggcg-3')and IV (SEQ ID NO:13) (5'-tgacttgagcgtcgatttttg-3') for the nonspecific one.

The other pair of control fragments (Control-Sss reference system) was developed for the experiments with SssI as well as HpaII and HhaII MTases. The specific probe contains a single unmodified recognition site for HhaI and HpaII MTases, and two recognition sites for SssI MTase, and therefore represents the unmethylated fraction of genomic DNA. The nonspecific fragment contains no target sites for HhaI, HpaII, or SssI MTases, and thus mimics the methylated fraction of genomic DNA. Both DNA probes were prepared by PCR amplification of mouse genomic DNA (cell line C57BL/6J) with two sets of primers: V (SEQ ID NO:14) (5'-gtgttggggtgactattatg-3') and VI (SEQ ID NO:15) (5'-cctatactcagcgcatcc-3') for the specific probe, and VII (SEQ ID NO:16) (5'-gcccacttcacttcttgtg-3') and VIII (SEQ ID NO:17) (5'-aggccaaaagaaagaagagat -3') for the nonspecific one. Quantitative assessments of each of the reference system are performed using our developed multiplex real-time PCR system (see below).

Pilot labeling experiment with M.HpaII MTase is performed as follows: the reaction mixture contains 1 µg of Control-H reference system, in which two control fragments were mixed at ratio 1:1, 4 µl or 10 µl of freshly diluted 1 mM Ado-11-amine cofactor, 10 µl of reaction buffer 50 mM Tris-HCl pH 7.4, 0.5 mM EDTA, 10 µl 2mg/ml BSA (0.2 mg/ml final concentration), 228 nM M.HpaII Q104A/N335A mutant and nuclease-free water to 100 µl of total reaction volume. After incubation at 37°C for 2 hours, M.HpaII is inactivated by heating for 15 min at 65°C.

For mTAG labeling of genomic DNA with M.HhaI, the following components were added into one tube: 500 ng of sheared and blunt-ended human brain genomic DNA, 100 ng of Control-H reference system (50 ng of each control fragment), 0.5 µl of freshly diluted 1 mM Ado-11-amine cofactor analog (5 µM final concentration of racemate), 10 µl of reaction buffer 50 mM Tris-HCl pH 7.4, 0.5 mM EDTA, 10 µl 2mg/ml BSA (0.2 mg/ml final concentration), 4 nM M.HhaI Q82/Y254S/N304A mutant and nuclease-free water to 100 µl of total reaction volume. After incubation at 37°C for 30 min, M.HhaI is inactivated by heating for 15 min at 65°C.

Genomic DNA labeling with M.SssI MTase is controlled with the Control-Sss reference system. The components of a labeling reaction: 300 ng sheared and blunt-ended genomic DNA of human brain, 50 ng of Control-Sss reference system (25 ng of each fragment), 2.5 µl of SssI reaction buffer 10 mM Tris-HCl pH 7.6, 50 mM NaCl, 0.1 mg/ml, 1.25 µl of freshly diluted 1 mM cofactor Ado-6-amine (50 µM final concentration of racemate), 1450 nM of M.SssI-His6 Q142A/N370A, and nuclease-free water to 25 µl of total reaction volume. After incubation at 37°C for 30 min, M.SssI enzyme is inactivated by heating for 15 min at 65°C.

After labeling, DNA samples are purified with Nucleotide Removal kit (Qiagen) using 10 V of PN buffer.

### 3) Attachment of biotin reporter to the terminal amino groups.

The resulting aminoderivatized DNA is combined in 0.15 M sodium bicarbonate (pH 9.0) buffer with 20 µl of 25 mg/ml freshly prepared dimethylformamide solution of (2-[Biotinamido]ethylamido)-3,3'-dithiodipropionic acid N-hydroxysuccinimide ester (Biotin-SS-NHS) (Sigma, cat. B4531) and the reaction incubated at room temperature for 2 h.. After reaction, DNA samples are purified with Nucleotide Removal kit (Qiagen) and eluted of the columns with 32 µl of EB buffer (10 mM Tris-HCl pH 8.5).

### 4) Affinity capture of labeled fragments on streptavidin-coated beads.

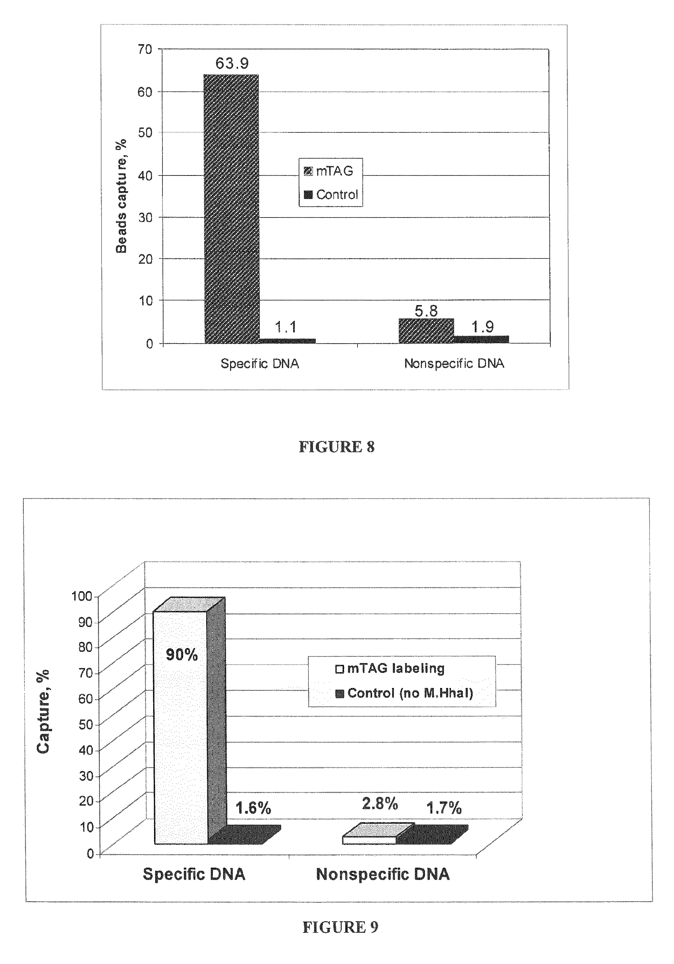

0.2 mg Dynabeads M-280 Streptavidin (Invitrogen) is collected on a magnet, the supernatant carefully removed and beads are washed with EB solution. After washing, the Dynabeads are settled on a magnet and resuspended in 8 µl of 5 M NaCl. The suspension is added to the DNA (32 µl) recovered in step 3). 40 µl of the resulting mixture in a final concentration of 1M NaCl is incubated at room temperature for 3 hours on a roller to keep the Dynabeads in suspension. The beads are then collected with a magnetic rack, washed three times with 40 µl of Washing buffer (10 mM Tris-HCl (pH 8.5), 3 M NaCl); twice with 40 µl of 7.5 mM sodium citrate (pH 7.0), 75 mM NaCl; twice with EB buffer, and finally re-suspended in 40 µl of 1 M Tris-HCI pH 7.4. On-beads DNA samples were immediately used for quantitation by multiplex real-time PCR on a Rotor-GeneTM 6000 real-time PCR instrument (Corbett Research) using Maxima^{™} Probe qPCR Master Mix (Fermentas). 0.25 µM of the respective dual-labeled probe (Metabion) and optimal amount of primers (Metabion) for the specific and the nonspecific DNA fragment were used in each reaction in a final volume of 25 µl (see table below for primer concentration and their sequence details in qPCR reaction). The amplification program was set as: 95°C for 10 min, 40 cycles 95°C for 15 s, 60°C for 1 min. Data were analyzed by Rotor-Gene^{™} software and reported as percentage of the material used in the step 4) Figures 8 to 10.

**Table 1. Primers and probes for quantification of Control-H reference system.**

| **Fragments** | **Primer** | **Primer sequence (5'→3')** | **Primer concentration in a multiplex qPCR reaction** |
|---|---|---|---|
| Specific | Specific-dir | gggttgccttactggttagc SEQ ID No: 18 | 0.9 µM |
| | Specific-rev | tccgcgtttccagactttac SEQ ID No: 19 | 0.9 µM |
| | TaqMan probe | FAM-atgaatcaccgatacgcgagcga-BHQ1 SEQ ID No:20 | 0.25 µM |
| Nonspecific | Nonspecific-dir | agctcactcaaaggcggtaa SEQ ID No:21 | 0.3 µM |
| | Nonspecific-rev | tttttgtgatgctcgtcagg SEQ ID No:22 | 0.3 µM |
| | TaqMan probe | HEX-aaggccaggaaccgtaaaaaggcc-BHQ1 SEQ ID No:23 | 0.25 µM |

**Table 2. Primers and probes for quantification of Control-Sss reference system.**

| **Fragment** | **Primer** | **Primer sequence (5'→3')** | **Primer concentration in a multiplex qPCR reaction** |
|---|---|---|---|
| Specific | Specific-dir | atgtgttggagtgtgcctga SEQ ID No:24 | 0.3 µM |
| | Specific-rev | gtggctctgattgatggctc SEQ ID No:25 | 0.3 µM |
| | TaqMan probe | FAM-tccctgtgtgatcacccctatgcttg-BHQ1 SEQ ID No:26 | 0.25 µM |
| Nonspecific | Nonspecific-dir | caggcctcttcaagggtca SEQ ID No:27 | 1µM |
| | Nonspecific-rev | aagagatgagggcctggg SEQ ID No:28 | 1µM |
| | TaqMan probe | JOE-tggcccatacctcttcaagggca-BHQ1 SEQ ID No:29 | 0.25 µM |

Figures 8 to 10 demonstrate the mTAG labeling efficiency of DNA fragments. An appropriate reference system (see below) alone or in the mixture with sonicated genomic DNA fragments was mTAG labeled with corresponding MTase. The resulting aminoderivatized DNA was treated with biotin disulfide N-hydroxysuccinimide ester (Sigma) and biotinylated DNA was separated on streptavidin-coated magnetic beads as described above. On-beads DNA samples were immediately used for quantitation by multiplex real-time PCR on a Rotor-Gene^{™} 6000 real-time PCR instrument (Corbett Research) using Maxima^{™} Probe qPCR Master Mix (Fermentas). Data were analyzed by Rotor-Gene^{™} software and reported as percentage of the material used for bead separation.

Figure 8 shows the HpaII-labeling and the capture on beads of the reference DNA system Control-H. The experiments with M.HpaII Q104A/N335A show that the unmethylated probe is recovered with the yield of ~50-60%, whereas the nonspecific probe is found at the level of 5-6%. While the labeling efficiency was good enough for analysis of labeled fragments on microarrays, quite high non-specific labeling required further optimization experiments. M.HpaII was excluded from further optimization due to its relatively poor specificity when discriminating specific versus non-specific target sites.

Figure 9 demonstrates the HhaI-labeling and enrichment efficiency of genomic DNA. 100 ng of Control-H was mixed with 500 ng of sonicated genomic DNA of human brain and labeled with HhaI Q82/Y254S/N304A as described above. The efficiency of labeling and capture on beads of genomic DNA is assessed by real-time analysis of the reference DNA fragments. After many labeling/enrichment procedures with HhaI MTase, its non-specific reaction was decreased to the level of 2.5%, while the selected labeling conditions gave the labeling of DNA fragment with one HhaI target site with the yield of ~70%.

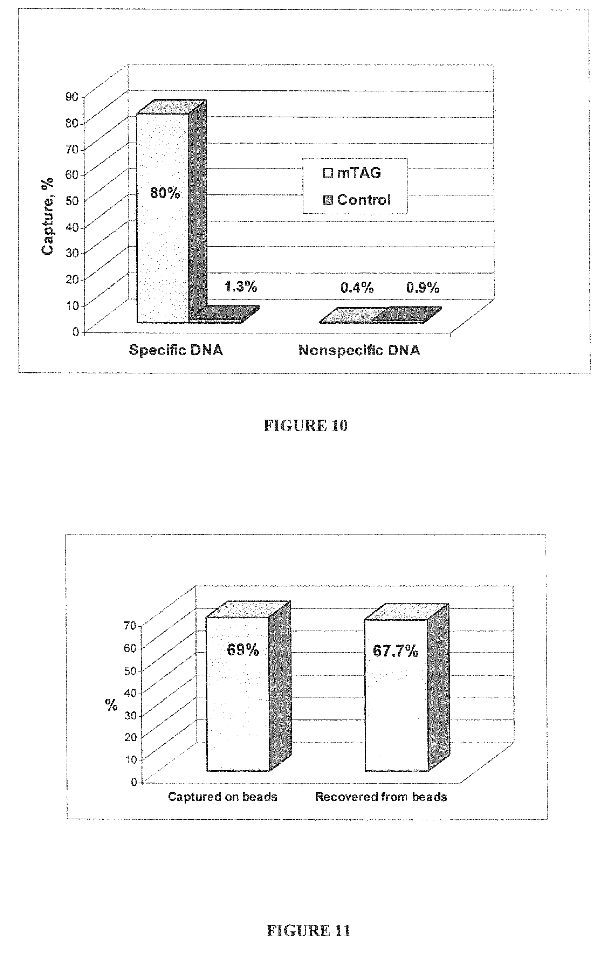

Figure 10 shows the SssI-labeling and enrichment efficiency of genomic DNA. 50 ng of Control-Sss reference system was mixed with 300 ng of sonicated genomic DNA of human brain and labeled with SssI Q142A/N370A as described above. The efficiency of labeling and capture on beads of genomic DNA is assessed by real-time analysis of the reference DNA fragments. The figure demonstrates that the specific probe containing two SssI target sites is captured with the yield of ~80%, whereas the nonspecific probe is found at the level of less than 1%.

### 5) Recovery of captured DNA.

Dithiothreitol (DTT) is used to cleave the disulfide bond present in the side chain of the biotin conjugate. For this, 2 M DTT stock is added to the suspension of DNA captured on beads (Step 4) to a final concentration of 200 mM and incubated at room temperature for one hour on a roller. Recovered DNA solution is collected from the beads with a magnetic rack. The DNA is supplemented with 0.1 volume of 3 M sodium acetate pH 7.0 and 1 volume of propanol-2, and incubated at -20°C overnight. The samples are then centrifuged at 20,000 x g for 30 min at 4°C, pellet washed with 200 µl of cold 75% ethanol, and centrifuged again for 15 min at the same conditions. DNA pellet is re-suspended in 9 µl of 1x T4 DNA Ligase buffer (40 mM Tris-HCl (pH 7.8 at 25°C), 10 mM MgCl₂, 10 mM DTT, 0.5 mM ATP).

Figure 11 shows the recovery of the captured mTAG labeled DNA from streptavidin coated magnetic beads. To this end, DTT is added to the suspension of DNA captured on beads (Step 4) to a final concentration of 200 mM, and the suspension is incubated at room temperature for one hour on a roller. The efficiency of recovery is tested by real-time PCR.

### 6) PCR amplification of the enriched DNA for microarray analysis.

PCR adaptors are prepared by mixing equal amounts (100 µM) of single-stranded oligonucleotides IX (SEQ ID NO:30) (5'-agttacatcttgtagtcagtctcca-3') and X (SEQ ID NO:31) (5'-tggagactgactacaagat-3') in 1x T4 DNA Ligase buffer (Fermentas), heating at 95°C for 5 min and cooling slowly to room temperature. To ligate adaptors to genomic DNA fragments, DNA recovered from beads in step 5) is incubated with 1 µl (5 µM) adaptor at 45°C for 10 min, the mixture is chilled on ice and after addition of 1 µl (5u) of T4 DNA Ligase (Fermentas) is further incubated at 22°C overnight.

For PCR amplification, 10 µl of the DNA sample are incubated with 100 mM 2-mercaptoethanol for 10 min at room temperature (to preclude the inadvertent formation of inter-nucleotide disulfide cross-links), followed by addition of the following PCR reagents (Fermentas): 10 µl of 10x Taq Buffer with (NH₄)₂SO₄, 10 µl of 2 mM dNTP (0.2 mM final concentration), 4 µl 25 mM MgCl₂ (1 mM final concentration), 1 µl IX (SEQ ID NO:30) oligonucleotide 100 µM (1µM final concentration), 1 µl (5 u) Taq DNA Polymerase (Fermentas), and nuclease-free water to 100 µl. PCR amplification is performed using the following cycling conditions: 1 min 50°C, 5 min 72°C, 4 min 94°C, 15 cycles of 1 min 94°C, 1 min 65°C, 1 min 72°C, and the final extension step is at 72°C for 2 min. The generated amplicons may be used in additional rounds of PCR amplification to generate desired amounts of DNA for microarray analysis.

### 7) Microarray analysis.

To validate the suitability of our method for genome-wide methylation analysis, DNA samples from human lung fibroblasts IMR90 were prepared according to the above procedure and were analyzed on an Affymetrix Human Tilling microarray 2.0R/D, which covers chromosomes 4, 15, 18. A series of labeling intensities were used to achieve optimal resolution of analysis DNA regions with various densities of CpG dinucleotides were labeled with different efficiencies. Labeling/enrichment procedure was optimized so that the control DNA fragment with two SssI target sites is recovered with the yield of 0%, 25%, or 80%. The first labeling condition (0%) tests the non-specific labeling and is the control sample, when labeling/enrichment reaction is done without methyltransferase.

The mTAG DNA samples were second-round amplified with 200 pmol of oligodeoxyribonucleotide IX (SEQ ID NO: 30), and the 20 mM dUTP was included in the dNTP mix as specified by Affymetrix. The PCR amplifications were performed at 95°C for 1 min followed by 15 cycles of 94°C for 15 seconds, 65°C for 15 seconds and 1 min at 72°C, with an extension of 5 seconds at last step of each subsequent cycle. The amplicons were purified using QIAquick PCR Purification Kit (Qiagen) and checked for quality and quantity on a NanoDrop 2000 spectrophotometer (Thermo Scientific).

In parallel with mTAG samples, methyl-DNA immunoprecipitation analysis (MeDIP, Weber et al., Nat Genet, 2005, 37, 853-62) was performed with the same genomic DNA. Two replicates of meDIP samples were prepared using MagMeDIP kit (Diagenode) according the manufacturer's instructions. An aliquot of each sample was used as template in two independent PCR reactions to confirm enrichment for methylated and de-enrichment for unmethylated sequences, compared to input DNA (sonicated DNA). The meDIP samples were further whole-genome amplified with the help of WGA kit (Sigma) which allows incorporation of dUTP, and prepared for hybridization on microarrays (see below).

For array hybridization, nine micrograms of PCR amplicons were fragmented to 50-100 bp using uracil DNA glycosylase enzyme, which cleaves DNA at incorporated dUTP (GeneChip® WT Double-Stranded DNA Terminal Labeling Kit, Affymetrix). Fragments were end-labeled according to the manufacturers' instructions. Prior to labeling, 1 µL of fragmented DNA was analyzed on a Bioanalyzer using DNA1000 Nano Chip (Agilent Technologies) to check the uniformity of the fragmented products. Individual samples were hybridized on a separate Gene Chip Human Tiling 2.0R Array for 16 h at 45°C. The arrays were washed, stained and scanned using an Affymetrix GeneChip Scanner as described in the Affymetrix Chromatin Immunoprecipitation Assay protocol.

Array data was quantile normalized and mTAG log rations for 0%-25% and 0%-80% probes were generated. For the analysis, relevant genomic regions were divided in tiles of the size 1 kb, and mean log-ratios of the probes in the tiles are calculated. Data was correlated with the bisulfitome data (minimum 5 reads) reported in Lister et al Nature, 2009, 462, 315-322 (http://neomorph.salk.edu/human methylome/data.html).

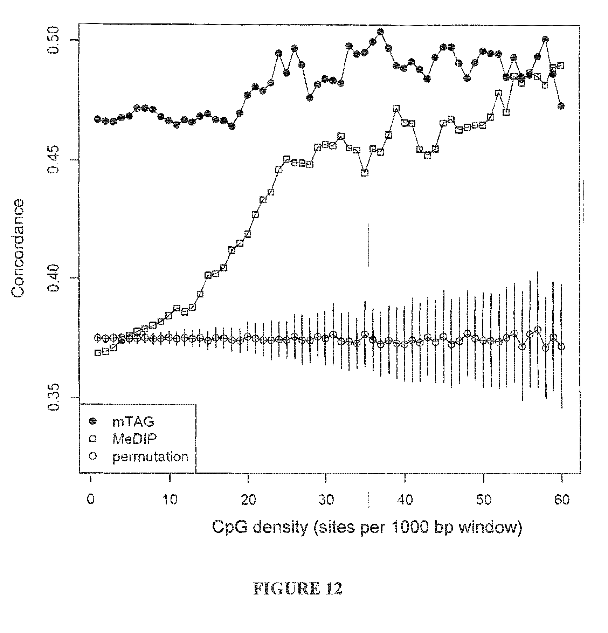

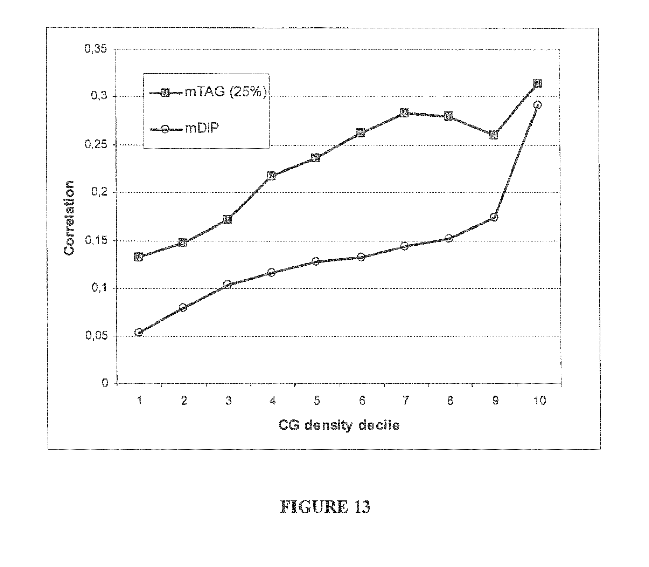

The results are shown in Figures 12 and 13. In particular, Figure 12 shows the concordance of the mTAG and meDIP data with the bisulfitome results (http://neomorph.salk.edu/human methylome/data.html) in human chromosome 15. For all types of data, mean log-ratios of the probes in the tiles are calculated and then attributed to one of the three methylation levels as follows: Weak methylation when signal is <25% of the signal distribution; Partial methylation when 25% < signal < 75% of the signal distribution; High methylation when signal is >75% of the signal distribution. The concordance results are averaged for tiles with identical number of CpG sites. The permutation result shows that the concordance with bisulfitome is around 0.375 when the calls are randomly made.

Figure 13 shows Pearson correlations of mTAG-based (labeling efficiency of 25%) analysis and meDIP based analysis of methylation across 10 deciles of CG density with the bisulfitome data in human chromosome 4 (Lister et al., Nature, 2009, 462,315-322)

The presented results thus show that mTAG enrichment is superior over MeDIP in regions of low to medium high CG content and is comparable to MeDIP in high CG content regions

From the examples described herein, one skilled in the art can easily ascertain the essential principles of this invention and without departing from the spirit and scope thereof, can make various modifications and changes of the invention in adapting to specific uses and conditions.

## Claims

1. A method for labeling unmethylated CpG dinucleotides within a DNA fragment, said method comprising the steps of:
(a)
(i) modifying the DNA fragment at the unmethylated CpG dinucleotide by contacting the DNA fragment with a mutant C5-methyltransferase enzyme and a co-factor under conditions which allow for the transfer of a part of the co-factor onto the unmethylated CpG dinucleotide to form a modified CpG dinucleotide; and
(ii) contacting the modified CpG dinucleotide with a compound comprising a label under conditions which allow for the transfer of the label to the modified CpG dinucleotide to form a labeled DNA fragment; or
(b) modifying the DNA fragment at the unmethylated CpG dinucleotide by contacting the DNA fragment with a mutant C5-methyltransferase enzyme and a co-factor comprising a label under conditions which allow for the transfer of the label onto the unmethylated CpG dinucleotide to form a labeled DNA fragment,
wherein the mutant C-5 methyltransferase enzyme has an amino acid sequence which comprises a glycine, serine, threonine, asparagine, alanine or valine in place of the conserved glutamine residue in motif IV and a glycine, serine, threonine, alanine or valine in place of the conserved asparagine residue in motif X,
and wherein, when the mutant C-5 methyltransferase enzyme comprises M.HhaI having an amino acid sequence which comprises the mutations Q32A and N304A, the DNA fragment is labeled using more than one mutant C-5 methyltransferase enzymes.

2. A method for labeling according to claim 1:
(i) wherein the mutant C5-methyltransferase enzyme comprises M.SssI having an amino acid sequence which comprises the mutations Q142A and N370A, or the mutant C5-methyltransferase enzyme comprises M.HpaII having an amino acid sequence which comprises the mutations Q104A and N335A; and/or
(ii) wherein (a) or (b) of claim 1 are repeated with at least one other mutant C-5 methyltransferase enzyme, and preferably wherein at least one other co-factor is used in the repeated step; and/or
(iii) wherein the DNA fragment is labeled using M.SssI having an amino acid sequence which comprises the mutations Q142A and N370A, M.HpaII having an amino acid sequence which comprises the mutations Q104A and N335A, and M.HhaI having an amino acid sequence which comprises the mutations Q82A, Y254S and N304A; and/or
(iv) wherein the label is an affinity tag, and preferably wherein the affinity tag is selected from c-myc-tag, HA-tag, digoxygenin, flag-tag, dinitrophenol, His tag, biotin, strep-tag, glutathione, nickel-nitrilotriacetic acid (NTA), an oligonucleotide primer, a DNA aptamer, an RNA aptamer or maltose.

3. A method for labeling according to claim 1 or claim 2 wherein the co-factor and/or the at least one other co-factor is represented by formula (I): 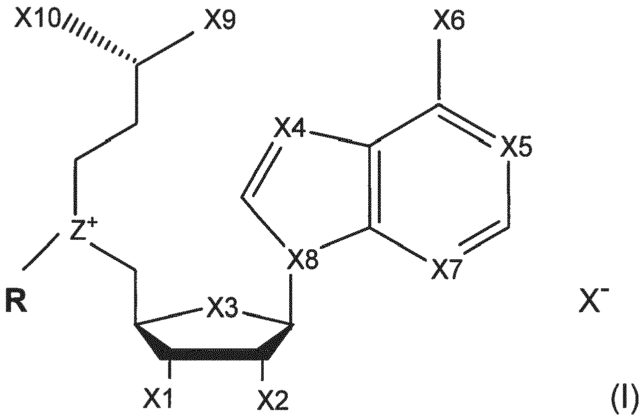 where
X1 and X2 represent -OH, -NH₂, -SH, -H or -F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH, -OCH₃, -H, -F, -Cl, -SH or -NHCH₃, and preferably is - NH₂; X9 represents -CO₂H, -PO₃H, -H, -CHO, -CH3, or -CH₂OH, and preferably is -CO₂H;
X10 represents -NH₂, -OH, -H, -CH₃, or -NHCH₃, and preferably is -NH₂;
X⁻ is an organic or inorganic anion selected from trifluoroacetate, formate, halide and sulfonate; Z represents S or Se, and preferably is S;
C-bound H atoms in the adenosine moiety can be replaced by -F, -OH, -NH₂, or -CH₃, but are preferably H;
R comprises -CH=CH- or -C=C- in a β-position to Z+ centre and separated therefrom by CR1R2-, where R1 and R2 are independently H or D;
R further comprises a functional group selected from an amino group, a thiol group, a 1,2-diol group, a hydrazine group, a hydroxylamine group, a 1,2-aminothiol group, an azide group, a diene group, an alkyne group, an arylhalide group, a terminal silylalkyne group, an N-hydroxysuccinimidyl ester group, a thioester group, an isothiocyanate group, an imidoester group, a maleimide group, a haloacetamide group, an aziridine group, an arylboronic acid group, an aldehyde group, a ketone group, a phosphane ester group, a dienophile group, and a terminal haloalkyne group,
more preferably:
(a) wherein the distance between -CH=CH- or -C=C- in the β-position to Z+ centre and the nearest electronegative atom or group in R is at least 2 carbon atoms; and/or
(b) wherein the nearest electronegative atom is selected from N, O, S, Br, Cl, F or Se; and/or
(c) wherein the functional group is a terminal functional group or a terminal protected functional group; and/or
(d) wherein the functional group is amino group, a thiol group, a 1,2-diol group, a hydroxylamine group, an azide group, a diene group, a terminal alkyne group, an arylhalide group, a maleimide group, an arylboronic acid group, an aldehyde group, a ketone group or a dienophile group, preferably wherein the functional group is an amino group; and/or
(e) wherein R comprises -C=C- in the β-position to Z+ centre and is separated therefrom by - CH₂-; and/or
(f) wherein R has the formula -CH₂C≡C(CH₂)₃NH₂ or -CH₂C≡C(CH₂)₃NHCO(CH₂)₃NH₂.

4. Use of a method for labeling according to any one of claims 1 to 3 in a method of DNA methylation analysis, and preferably: (i) in a method of genomic DNA methylation profiling; or (ii) in a method for analyzing unmethylated CpG dinucleotides within one or more DNA molecules comprising the steps of:
(a) providing fragments of the DNA molecules;
(b) labeling the unmethylated CpG dinucleotides according to any one of claims 1 to 3 to produce labeled DNA fragments;
(c) enriching the labeled DNA fragments;
(d) amplifying the enriched labeled DNA fragments; and
(e) analyzing the amplified DNA fragments to determine the methylation status of the CpG dinucleotides.

5. Use according to claim 4: (i) wherein the fragments of step (a) are formed by enzymatic, chemical or mechanical digestion of the one or more DNA molecules, and preferably are formed by DNA shearing; and/or
(ii) which further comprises a step prior to step (d) of ligating an adaptor to the 5' and the 3' end of each fragment, wherein the adaptor comprises a nucleic acid sequence capable of hybridizing with a primer for a polymerase chain reaction; and/or
(iii) wherein step (c) comprises affinity capture of labeled fragments on beads and recovery of the captured labeled DNA from the beads, and preferably wherein step (b) comprises labeling with biotin and step (c) comprises affinity capture of labeled fragments on streptavidin-coated beads and recovery of the captured labeled DNA from the beads; and/or
(iv) wherein step (e) comprises analyzing the labeled DNA fragments on a tiling microarray.

6. A mutant CpG C-5 methyltransferase enzyme, said enzyme having an amino acid sequence which comprises a glycine, serine, threonine, asparagine, alanine or valine in place of the conserved glutamine residue in motif IV and a glycine, serine, threonine, alanine or valine in place of the conserved asparagine residue in motif X, wherein said enzyme is not M.HhaI.

7. A mutant CpG C-5 methyltransferase enzyme according to claim 6:
(a) which is an M.SssI enzyme having an amino acid sequence which comprises the mutations at conserved residues Q142 and N370, and preferably wherein:
(i) the mutant M.SssI enzyme comprises the mutations Q142A and N370A, and preferably wherein the mutant M.SssI enzyme also has an amino acid sequence which is at least 85% identical to SEQ ID No: 1; or
(ii) the mutant M.SssI enzyme has an amino acid sequence which comprises SEQ ID No: 2 and SEQ ID No: 3, and preferably wherein the mutant M.SssI enzyme also has an amino acid sequence which is at least 85% identical to SEQ ID No: 1; or
(iii) the mutant M.SssI enzyme has an amino acid sequence which is at least 85% identical to SEQ ID No: 1,
or (b) which is M.HpaII enzyme having an amino acid sequence which comprises the mutations at conserved residues Q104 and N335, and preferably wherein:
(i) the mutant M.HpaII enzyme comprises the mutations Q104A and N335A, and preferably wherein the mutant M.HpaII enzyme also has an amino acid sequence which is at least 85% identical to SEQ ID No: 4; or
(ii) the mutant M.HpaII enzyme has an amino acid sequence which comprises SEQ ID No: 5 and SEQ ID No: 6, and preferably wherein the mutant M.HpaII enzyme also has an amino acid sequence which is at least 85% identical to SEQ ID No: 4; or
(iii) the mutant M.HpaII enzyme has an amino acid sequence which is at least 85% identical to SEQ ID No: 4.

8. A polynucleotide which encodes the CpG methyltransferase of claim 6 or claim 7.

9. A compound represented by formula (I): 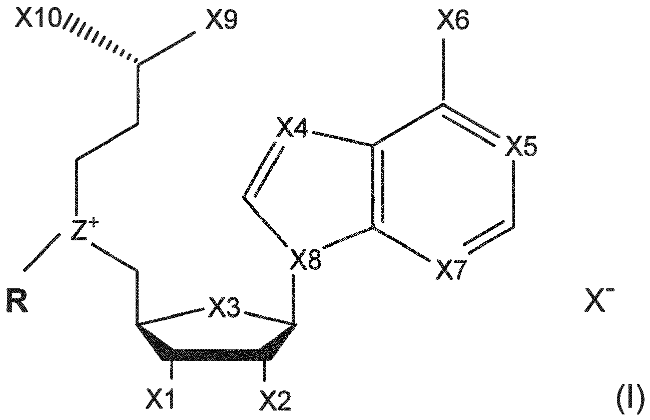 where
X1 and X2 represent -OH, -NH₂, -SH, -H or -F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH, -OCH₃, -H, -F, -Cl, -SH or -NHCH₃, and preferably is - NH₂;
X9 represents -CO₂H, -PO₃H, -H, -CHO, -CH₃, or -CH₂OH, and preferably is -CO₂H;
X10 represents -NH₂, -OH, -H, -CH₃, or -NHCH₃, and preferably is -NH₂;
X- is an organic or inorganic anion selected from trifluoroacetate, formate, halide and sulfonate; Z represents S or Se, and preferably is S;
C-bound H atoms in the adenosine moiety can be replaced by -F, -OH, -NH₂, or -CH₃, but are preferably H;
R comprises -CH=CH- or -C=C- in a β-position to Z+ centre and separated therefrom by CR1R2-, where R1 and R2 are independently H or D;
R further comprises a functional group selected from an amino group, a thiol group, a 1,2-diol group, a hydrazine group, a hydroxylamine group, a 1,2-aminothiol group, an azide group, a diene group, an alkyne group, an arylhalide group, a terminal silylalkyne group, an N-hydroxysuccinimidyl ester group, a thioester group, an isothiocyanate group, an imidoester group, a maleimide group, a haloacetamide group, an aziridine group, an arylboronic acid group, an aldehyde group, a ketone group, a phosphane ester group, a dienophile group, and a terminal haloalkyne group,
wherein the distance between -CH=CH- or -C=C- in the β-position to Z+ centre and the functional group is no more than 7 atoms in length,
and wherein the distance between -CH=CH- or -C≡C- and the nearest electronegative atom or group in R is at least 2 carbon atoms.

10. A compound according to claim 9 wherein: (a) the nearest electronegative atom is selected from N, O, S, Br, Cl, F or Se; and or
(b) the functional group is a terminal functional group or a terminal protected functional group; and/or
(c) the nearest electronegative group is the functional group, and preferably wherein -CH=CH- or -C≡C- in the β-position to Z+ centre is separated from the functional group by two or three carbon units, more preferably wherein -CH=CH- or -C=C- in the β-position to Z+ centre is separated from the functional group by -CR3R4-CR5R6- or -CR3R4-CR5R6-CR7R8- and R3 to R8 are independently H or a C₁ -C₃ alkyl; and/or
(d) the functional group is an amino group, a thiol group, a 1,2-diol group, a hydroxylamine group, an azide group, a diene group, a terminal alkyne group, an arylhalide group, a maleimide group, an arylboronic acid group, an aldehyde group, a ketone group or a dienophile group, and preferably wherein the functional group is an amino group; and/or
(e) R comprises -C=C- in the β-position to Z+ centre and is separated therefrom by -CH₂-, preferably wherein R has the formula -CH₂C≡C(CH₂)₃NH₂.

11. A kit comprising: (a) at least two methyltransferase enzymes according to claim 6 or claim 7; or
(b) the compound of claim 9 or claim 10 and a methyltransferase enzyme, and preferably
(i) wherein the methyltransferase enzyme is a DNA methyltransferase; more preferably
(ii) wherein the DNA methyltransferase is a CpG C-5 methyltransferase enzyme; still more preferably
(iii) wherein the CpG C-5 methyltransferase enzyme is an enzyme according to any one of claims 25 to 33, or M.HhaI, wherein the M.HhaI comprises mutations at Q82 and N304, wherein Q82 is replaced by a glycine, serine, threonine, asparagine, alanine or valine, and N304 is replaced by a glycine, serine, threonine, alanine or valine; and most preferably
(iv) wherein the M.HhaI further comprises the mutation Y254S, and preferably also comprises the mutations Q82A and N304A.

12. A complex of a compound according to claim 9 or claim 10 and a methyltransferase with normally uses S-adenosyl-L-methionine (SAM or AdoMet) as a cofactor, and preferably:
(i) wherein said methyltransferase normally transfers the methyl residue of AdoMet onto a nucleic acid molecule, a polypeptide, a carbohydrate or a small molecule; and/or
(ii) wherein the methyltransferase is according to claim 6 or claim 7.

13. Use of a compound of claim 9 or claim 10, or an enzyme of claim 6 or claim 7, or a kit of claim 11 for modifying or labeling a target molecule, preferably wherein the target molecule is a DNA molecule.

14. A method of producing a compound according to claim 9 or claim 10 comprising a step of reacting an activated compound comprising R with a compound of formula IV: 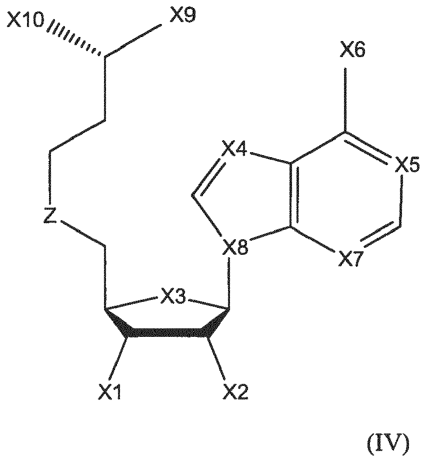 where
X1 and X2 represent -OH, -NH₂, -SH, -H or -F, and preferably is -OH;
X3 represents -O-, -NH-, -CH₂-, -S-, or -Se-, and preferably is -O;
X4, X5, X7, X8 represent -N-, or -CH-, and preferably is -N;
X6 represents -NH₂, -OH, -OCH₃, -H, -F, -Cl, -SH or -NHCH₃, and preferably is - NH₂;
X9 represents -CO₂H, -PO₃H, -H, -CHO, -CH3, or -CH₂OH, and preferably is -CO₂H;
X10 represents -NH₂, -OH, -H, -CH₃, or -NHCH₃, and preferably is -NH₂;
Z represents S or Se, and preferably is S;
C-bound H atoms in the adenosine moiety can be replaced by -F, -OH, -NH₂, or -CH₃, but are preferably H;
under conditions which allow the R group to be coupled to the Z of the compound of formula IV.

15. A method of producing a compound according to claim 14, wherein: (a) the activated compound comprising R is activated with an aryl sulfonate or an alkyl sulfonate containing from 1 to 3 electron-withdrawing groups, and preferably wherein the electron-withdrawing groups are selected from nitro, nitrile, halogen, carboxyl, sulphone or sulfate; and/or
(b) the activated compound comprising R further comprises a protective group attached to the functional group, and preferably wherein the protective group is *N*-BOC, 1-adamatyloxycarbonyl, trimethylsilylethyloxycarbonyl, nitrophenyloxycarbonyl, nitrophenylethyloxycarbonyl, or dimethoxynitrobenzyloxycarbonyl (DMNB); and/or
(c) the functional group is a primary amine, the method comprising the steps of:
i) protection of -NH₂ group and activation of -OH group in a compound represented by the formula (II) or the formula (III): 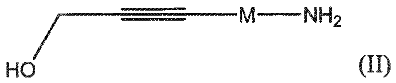 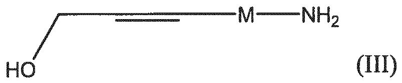 in which M is -CR3R4-CR5R6- or -CR3R4-CR5R6-CR7R8-, wherein R3 to R8 are independently H, D, or an alkyl group.
ii) reaction of the compound produced from step (i) represented by the formula (III) with a compound represented by the formula (IV):
(iii) deprotection of the protected -NH₂ group to form the compound according to claim 9,
and preferably:
(i) wherein the -NH₂ group is protected by reaction with the following compound: 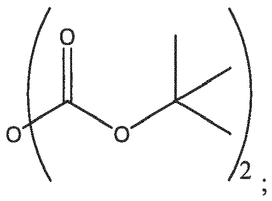 and/or
(ii) wherein the -OH group is activated by reaction with the following compound:

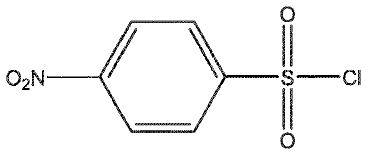

16. A method of producing a mutant CpG C-5 methyltransferase enzyme according to claim 6 or claim 7 comprising expressing the polynucleotide of claim 8.

17. A nucleic acid molecule comprising at least one residue in which a cytosine base is derivatised at position 5 with a group R, wherein R comprises -CR1R2-CH=CH- or -CR1R2-C=C-, where R1 and R2 are independently H or D, and wherein R further comprises a functional group selected from an amino group, a thiol group, a 1,2-diol group, a hydrazine group, a hydroxylamine group, a 1,2-aminothiol group, an azide group, a diene group, an alkyne group, an arylhalide group, a terminal silylalkyne group, an N-hydroxysuccinimidyl ester group, a thioester group, an isothiocyanate group, an imidoester group, a maleimide group, a haloacetamide group, an aziridine group, an arylboronic acid group, an aldehyde group, a ketone group, a phosphane ester group, a dienophile group and a terminal haloalkyne group,
wherein the distance between -CH=CH- or -C=C- and the functional group is no more than 7 atoms in length,
and wherein the distance between -CH=CH- or -C=C- and the nearest electronegative atom or group in R is at least 2 carbon atoms.

18. A nucleic acid molecule according to claim 17 wherein: (a) the nearest electronegative atom is selected from N, O, S, Br, Cl, F or Se; and/or
(b) the functional group is a terminal functional group or a terminal protected functional group; and/or
(c) the nearest electronegative group is the functional group; and/or
(d) -CH=CH- or -C=C- is separated from the functional group by two or three carbon units, and preferably wherein -CH=CH- or -C=C- is separated from the functional group by -CR3R4-CR5R6- or -CR3R4-CR5R6-CR7R8- and R3 to R8 are independently H or a C₁ -C₃ alkyl; and/or
(e) the functional group is an amino group, a thiol group, a 1,2-diol group, a hydroxylamine group, an azide group, a diene group, a terminal alkyne group, an arylhalide group, a maleimide group, an arylboronic acid group, an aldehyde group, a ketone group or a dienophile group, and preferably wherein the functional group is an amino group, more preferably wherein R is 6-aminohexyn-2-yl; and/or
(f) the nucleic acid molecule is DNA, and preferably wherein the nucleic acid molecule comprises at least one 5-(6-aminohexyn-2-yl)-2'-deoxycytidine.
